# EUROPEAN PATENT APPLICATION

(11) **EP 1 486 504 A1**
(43) Date of publication of application: **15.12.2004**
(21) Application number: 03705110.9
(22) Date of filing: 13.02.2003
(51) Int. Cl.: C07H 19/06, C07H 19/16, C07H 21/00, A61K 31/712

(54) **NUCLEOSIDE ANALOGUES WHOSE SUGAR MOIETIES ARE BOUND IN S-FORM AND OLIGONUCLEOTIDE DERIVATIVES COMPRISING NUCLEOTIDE ANALOGUES THEREOF**

(30) Priority: 13.02.2002 JP 2002035809; 19.09.2002 JP 2002272652
(71) Applicant: Imanishi, Takeshi, Nara-shi, Nara 631-0045 (JP)
(72) Inventor: IMANISHI, Takeshi, Nara-shi, Nara 631-0045 (JP); OBIKA, Satoshi, Takatsuki-shi, Osaka 569-1022 (JP)
(74) Representative: VOSSIUS & PARTNER
(86) International application number: PCT/JP2003/001486
(87) International publication number: WO 2003/068794

(57) **Abstract**

Compounds of the following general formula (1) and salts thereof: 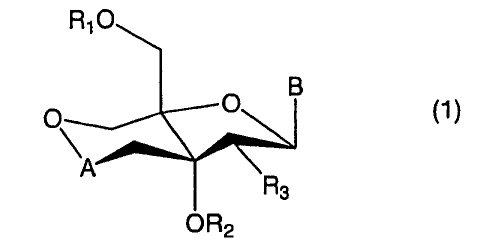 where A represents an alkylene group having 1 to 2 carbon atoms, etc.; B represents an aromatic heterocyclic group which may have a substituent, etc.; R₁ and R₂ each represent a hydrogen atom, a protective group for a hydroxyl group for synthesis of nucleic acid, a phosphate group, or -P(R₄)R₅ [where R₄ and R₅ are the same or different, and each represent a hydroxyl group, a hydroxyl group protected with a protective group for synthesis of nucleic acid, a mercapto group, a mercapto group protected with a protective group for synthesis of nucleic acid, etc.]; and R₃ represents a hydrogen atom, a halogen atom, a hydroxyl group, a hydroxyl group protected with a protective group for synthesis of nucleic acid, etc.

These compounds are useful for producing oligonucleotide analogues useful for the antisense method, antigene method, etc., and for producing their intermediates.

## Description

### [TECHNICAL FIELD]

This invention relates to oligonucleotide analogues which have stable and excellent antisense or antigene activity, or have excellent activity as drugs for detection of particular genes or primers for initiation of amplification of particular genes, and which are useful as materials for various physiologically active or bioactive substances and pharmaceuticals, as functional materials for RNAi and decoy double-stranded oligonucleotides, as functional materials for DNA chips and molecular beacons targeting single-stranded nucleic acids such as cDNA, as functional materials for uses in various antisense methods (including ribozymes and DNAzymes) or antigene methods, and as materials for high sensitivity analysis of in vivo trace components by combination with fluorescent or luminescent substances. The invention also relates to nucleoside analogues, which are intermediates for production of the oligonucleotide analogues.

### [BACKGROUND ART]

In 1978, it was reported for the first time that antisense molecules inhibited infection by influenza virus. Since then, reports have been issued that antisense molecules also inhibited oncogene expression and AIDS infection. Since antisense oligonucleotides specifically control the expression of undesirable genes, they have become one of the most promising fields as medicines in recent years.

The antisense method is based on the concept of controlling a series of information flow steps of the so-called central dogma, DNA → mRNA → protein, by use of an antisense oligonucleotide.

When a natural type oligonucleotide was applied as an antisense molecule to this method, however, problems arose such that it underwent hydrolysis by enzymes present in vivo, and its cell membrane permeation was not high. To resolve these problems, numerous nucleic acid derivatives have been synthesized, and have been extensively studied. For example, phosphorothioates having an oxygen atom on a phosphorus atom substituted by a sulfur atom, and methylphosphonates having an oxygen atom on a phosphorus atom substituted by a methyl group were synthesized. Recently, the derivatives having the phosphorus atom also substituted by a carbon atom, and molecules having ribose converted to an acyclic skeleton have also been synthesized (F. Eckstein et al., Biochem., 18, 592 (1979), P.S. Miller et al., Nucleic Acids Res., 11, 5189 (1983), P. Herdewijn et al., J. Chem. Soc. Perkin Trans. 1, 1567 (1993), P.E. Nielsen et al., Science, 254, 1497 (1991)).

However, none of these derivatives are fully satisfactory in in vivo stability, ease of synthesis of oligonucleotides, and so on. Moreover, the sugar portion of nucleic acid usually has any of two conformations, N-form (C3'-endo type) and S-form (C2'-endo type) (Biochemistry, 13, 4417, 1974). By fixing the conformation of the sugar portion in one of these forms, an entropy change during hybrid formation can be decreased, and an improvement in binding affinity for target nucleic acid can be expected.

Actually, 2'-O,4'-methylene bridged nucleic acid, etc. having the conformation of the sugar portion fixed in N-form have been known, and improvements in double strand and triple strand forming capacity for target nucleic acid have been reported (Tetrahedron Lett., 38, 8735, 1997; Japanese Patent Application Laid-Open No. 1998-304889). However, there have been few reports of nucleic acid analogues having the conformation of the sugar portion fixed in S-form, and S-form nucleic acid analogues capable of forming double strands have not been reported.

In the light of the above-described conventional technologies, provision is desired of nucleotide analogues which have high cell membrane permeation under in vivo conditions, which are minimally hydrolyzed with enzymes, whose synthesis is easy, and which are useful for the antisense method, the antigene method, RNAi, and the decoy method.

### [DISCLOSURE OF THE INVENTION]

We, the inventors of the present invention, found nucleic acid derivatives with a modified sugar portion of nucleic acid, which may be useful as materials for various physiologically active or bioactive substances and pharmaceuticals, as functional materials for RNAi (Nature, Vol. 411, 494-498, 2001) and decoy double-stranded oligonucleotides, as functional materials for DNA chips and molecular beacons targeting single-stranded nucleic acids such as cDNA, as functional materials for uses in various antisense methods (including ribozymes and DNAzymes) or antigene methods, and as materials for high sensitivity analysis of in vivo trace components by combination with fluorescent or luminescent substances; and which are nucleotide analogues containing nucleoside analogues having the conformation of the sugar portion fixed in S-form. Based on this finding, we accomplished the present invention. The present invention will be described hereinbelow.

### [EMBODIMENTS OF THE INVENTION]

Nucleoside analogues of the present invention are compounds of the following general formula (1) and their salts: 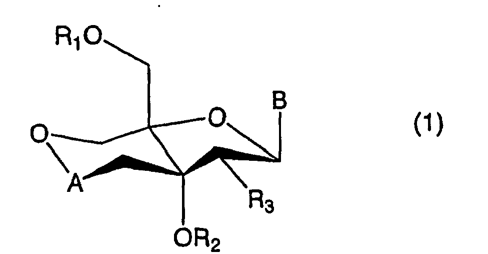 Where A represents an alkylene group having 1 to 2 carbon atoms, or -CH₂-O- (where the oxygen atom is linked to the methylene group at the 3'-position),
B represents an aromatic heterocyclic group or an aromatic hydrocarbon ring group which may have a substituent,
R₁ and R₂ are the same or different, and each represent a hydrogen atom, a protective group for a hydroxyl group for synthesis of nucleic acid, an alkyl group, an alkenyl group, a cycloalkyl group, an aryl group, an aralkyl group, an acyl group, a silyl group, a phosphate group, a phosphate group protected with a protective group for synthesis of nucleic acid, or -P(R₄)R₅ [where R₄ and R₅ are the same or different, and each represent a hydroxyl group, a hydroxyl group protected with a protective group for synthesis of nucleic acid, a mercapto group, a mercapto group protected with a protective group for synthesis of nucleic acid, an amino group, an alkoxy group having 1 to 5 carbon atoms, an alkylthio group having 1 to 5 carbon atoms, a cyanoalkoxy group having 1 to 6 carbon atoms, or an amino group substituted by an alkyl group having 1 to 5 carbon atoms], and
R₃ represents a hydrogen atom, a halogen atom, a hydroxyl group, a hydroxyl group protected with a protective group for synthesis of nucleic acid, a mercapto group, a mercapto group protected with a protective group for synthesis of nucleic acid, an amino group, an amino group protected with a protective group for synthesis of nucleic acid, an alkoxy group having 1 to 5 carbon atoms, an alkylthio group having 1 to 5 carbon atoms, a cyanoalkoxy group having 1 to 6 carbon atoms, or an amino group substituted by an alkyl group having 1 to 5 carbon atoms.

Nucleotide analogues of the present invention are oligonucleotide analogues containing one or more units having a structure represented by the following general formula (2), or pharmacologically acceptable salts of the oligonucleotide analogues, provided that if the oligonucleotide analogues or salts thereof contain two or more units having one or more of such structures, B is the same or different among these structures. 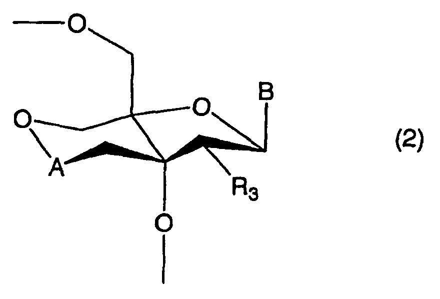 where A represents an alkylene group having 1 to 2 carbon atoms or -CH₂-O- (where the oxygen atom is linked to the methylene group at the 3'-position),
B represents an aromatic heterocyclic group or an aromatic hydrocarbon ring group which may have a substituent, and
R₃ represents a hydrogen atom, a halogen atom, a hydroxyl group, a hydroxyl group protected with a protective group for synthesis of nucleic acid, a mercapto group, a mercapto group protected with a protective group for synthesis of nucleic acid, an amino group, an amino group protected with a protective group for synthesis of nucleic acid, an alkoxy group having 1 to 5 carbon atoms, an alkylthio group having 1 to 5 carbon atoms, a cyanoalkoxy group having 1 to 6 carbon atoms, or an amino group substituted by an alkyl group having 1 to 5 carbon atoms.

In the present invention, examples of the "alkylene group having 1 to 2 carbon atoms" as A in the general formula (1) or (2) are methylene and ethylene groups. Preferred is a methylene group.

In the general formula (1) or (2), a methylene group is preferred as A.

In the general formula (1) or (2), the aromatic heterocyclic group as B refers to a structure in which the carbon atom, the constituent atom of a hydrocarbon ring, has been replaced by one or more heteroatoms such as nitrogen atoms, sulfur atoms or oxygen atoms. This structure represents any of groups having 5- to 20-membered rings and showing aromaticity, including a single ring and condensed rings. Concretely, the aromatic heterocyclic group includes, for example, pyrimidine or purine nucleic acid bases, and pyrimidine or purine nucleic acid bases which may have one or more substituents selected from the α group to be described below. The pyrimidine or purine nucleic acid bases include bases generally known as constituent elements of nucleic acids (for example, guanine, adenine, cytosine, thymine and uracil), and all chemical structures that can act as, or can be used instead of, other nucleic acid component bases similar to these bases. Others are also included, such as thiophene, thianthrene, furan, pyran, isobenzofuran, chromene, xanthene, phenoxthine, pyrrole, imidazole, pyrazole, isothiazole, isoxazole, pyridazine, indolizine, indole, isoindole, isoquinoline, quinoline, naphthyridine, quinoxaline, quinazoline, pteridine, carbazole, phenanthridine, acridine, perimidine, phenazine, phenarsazine, phenothiazine, brazan, phenoxazine, pyrrolidine, pyrroline, imidazolidine, imidazoline, and pyrazolidine. Preferably, the aromatic heterocyclic groups are the pyrimidine or purine nucleic acid bases, and pyrimidine or purine nucleic acid bases which may have one or more substituents selected from the α group to be described below. Concretely, the preferred one is a purin-9-yl group, a 2-oxo-pyrimidin-1-yl group, or a purin-9-yl group or a 2-oxo-pyrimidin-1-yl group which has a substituent selected from the following α group:
α group: A hydroxyl group, a hydroxyl group protected with a protective group for synthesis of nucleic acid, an alkoxy group having 1 to 5 carbon atoms, a mercapto group, a mercapto group protected with a protective group for synthesis of nucleic acid, an alkylthio group having 1 to 5 carbon atoms, an amino group, an amino group protected with a protective group for synthesis of nucleic acid, an amino group substituted by an alkyl group having 1 to 5 carbon atoms, an alkyl group having 1 to 5 carbon atoms, and a halogen atom.

Preferred as the "purine nucleic acid base which may have a substituent" is a 6-aminopurin-9-yl (i.e. adeninyl) group, a 6-aminopurin-9-yl group having an amino group protected with a protective group for synthesis of nucleic acid, a 2,6-diaminopurin-9-yl group, a 2-amino-6-chloropurin-9-yl group, a 2-amino-6-chloropurin-9-yl group having an amino group protected with a protective group for synthesis of nucleic acid, a 2-amino-6-fluoropurin-9-yl group, a 2-amino-6-fluoropurin-9-yl group having an amino group protected with a protective group for synthesis of nucleic acid, a 2-amino-6-bromopurin-9-yl group, a 2-amino-6-bromopurin-9-yl group having an amino group protected with a protective group for synthesis of nucleic acid, a 2-amino-6-hydroxypurin-9-yl (i.e. guaninyl) group, a 2-amino-6-hydroxypurin-9-yl group having an amino group protected with a protective group for synthesis of nucleic acid, a 6-amino-2-methoxypurin-9-yl group, a 6-amino-2-chloropurin-9-yl group, a 6-amino-2-fluoropurin-9-yl group, a 2,6-dimethoxypurin-9-yl group, a 2,6-dichloropurin-2-yl group, or a 6-mercaptopurin-9-yl group. More preferred is a 6-benzoylaminopurin-9-yl group, an adeninyl group, a 2-isobutyrylamino-6-hydroxypurin-9-yl group, or a guaninyl group.

Preferred as the "pyrimidine nucleic acid base which may have a substituent" is a 2-oxo-4-amino-1,2-dihydropyrimidin-1-yl (i.e. cytosinyl) group, a 2-oxo-4-amino-1,2-dihydropyrimidin-1-yl group having an amino group protected with a protective group for synthesis of nucleic acid, a 2-oxo-4-amino-5-fluoro-1,2-dihydropyrimidin-1-yl group, a 2-oxo-4-amino-5-fluoro-1,2-dihydropyrimidin-1-yl group having an amino group protected with a protective group for synthesis of nucleic acid, a 4-amino-2-oxo-5-chloro-1,2-dihydropyrimidin-1-yl group, a 2-oxo-4-methoxy-1,2-dihydropyrimidin-1-yl group, a 2-oxo-4-mercapto-1,2-dihydropyrimidin-1-yl group, a 2-oxo-4-hydroxy-1,2-dihydropyrimidin-1-yl (i.e. uracinyl) group, a 2-oxo-4-hydroxy-5-methyl-1,2-dihydropyrimidin-1-yl (i.e. thyminyl) group, or a 4-amino-5-methyl-2-oxo-1,2-dihydropyrimidin-1-yl (i.e. 5-methylcytosinyl) group. More preferred is a 2-oxo-4-benzoylamino-1,2-dihydropyrimidin-1-yl group, a cytosinyl group, a thyminyl group, a uracinyl group, a 2-oxo-4-benzoylamino-5-methyl-1,2-dihydropyrimidin-1-yl group, or a 5-methylcytosinyl group.

Of the "purine or pyrimidine nucleic acid bases which may have a substituent", still further preferred is a 6-aminopurin-9-yl (i.e. adeninyl) group, a 6-aminopurin-9-yl group having an amino group protected with a protective group for synthesis of nucleic acid, a 2,6-diaminopurin-9-yl group, a 2-amino-6-chloropurin-9-yl group, a 2-amino-6-chloropurin-9-yl group having an amino group protected with a protective group for synthesis of nucleic acid, a 2-amino-6-fluoropurin-9-yl group, a 2-amino-6-fluoropurin-9-yl group having an amino group protected with a protective group for synthesis of nucleic acid, a 2-amino-6-bromopurin-9-yl group, a 2-amino-6-bromopurin-9-yl group having an amino group protected with a protective group for synthesis of nucleic acid, a 2-amino-6-hydroxypurin-9-yl (i.e. guaninyl) group, a 2-amino-6-hydroxypurin-9-yl group having an amino group protected with a protective group for synthesis of nucleic acid, a 6-amino-2-methoxypurin-9-yl group, a 6-amino-2-chloropurin-9-yl group, a 6-amino-2-fluoropurin-9-yl group, a 2,6-dimethoxypurin-9-yl group, a 2,6-dichloropurin-9-yl group, or a 6-mercaptopurin-9-yl group, a 2-oxo-4-amino-1,2-dihydropyrimidin-1-yl (i.e. cytosinyl) group, a 2-oxo-4-amino-1,2-dihydropyrimidin-1-yl group having an amino group protected with a protective group for synthesis of nucleic acid, a 2-oxo-4-amino-5-fluoro-1,2-dihydropyrimidin-1-yl group, a 2-oxo-4-amino-5-fluoro-1,2-dihydropyrimidin-1-yl group having an amino group protected with a protective group for synthesis of nucleic acid, a 4-amino-2-oxo-5-chloro-1,2-dihydropyrimidin-1-yl group, a 2-oxo-4-methoxy-l,2-dihydropyrimidin-1-yl group, a 2-oxo-4-mercapto-1,2-dihydropyrimidin-1-yl group, a 2-oxo-4-hydroxy-1,2-dihydropyrimidin-1-yl (i.e. uracinyl) group, a 2-oxo-4-hydroxy-5-methyl-l,2-dihydropyrimidin-l-yl (i.e. thyminyl) group, or a 4-amino-5-methyl-2-oxo-1,Z-dihydropyrimidin-1-yl (i.e. 5-methylcytosinyl) group, or a 4-amino-5-methyl-2-oxo-1,2-dihydropyrimidin-1-yl having an amino group protected with a protective group for synthesis of nucleic acid.

In the general formula (1) or (2), the aromatic hydrocarbon ring group as B refers to a monovalent substituent remaining after removing one hydrogen atom from a hydrocarbon ring with 6 to 20 carbon atoms showing aromatic properties, and includes a single ring or condensed rings. Concretely, phenyl, indenyl, naphthyl, pentalenyl, azulenyl, heptalenyl, biphenylenyl, indacenyl, fluorenyl, phenanthryl, and anthryl, for example, are named. All structures that can be used instead as the base portion of the nucleic acid component in attaining the object of the present invention are also included as other examples. Moreover, the aromatic hydrocarbon ring may be substituted by one or more groups among a hydroxyl group, a hydroxyl group protected with a protective group for synthesis of nucleic acid, an amino group, an amino group protected with a protective group for synthesis of nucleic acid, a halogen atom, a lower alkyl group, an alkoxy group, a carboxyl group, an aryloxy group, a nitro group, a trifluoromethyl group, and a phenyl group. Examples of such an optionally substituted aromatic hydrocarbon group are 4-hydroxyphenyl, 2-hydroxyphenyl, 4-aminophenyl, 2-aminophenyl, 2-methylphenyl, 2,6-dimethylphenyl, 2-chlorophenyl, 4-chlorophenyl, 2,4-dichlorophenyl, 2,5-dichlorophenyl, 2-bromophenyl, 4-methoxyphenyl, 4-chloro-2-nitrophenyl, 4-nitrophenyl, 2,4-dinitrophenyl, and biphenyl. Preferred examples of the optionally substituted aromatic hydrocarbon ring group are a phenyl group, and a phenyl group substituted by a hydroxyl group, a hydroxyl group protected with a protective group for synthesis of nucleic acid, an amino group, an amino group protected with a protective group for synthesis of nucleic acid, a lower alkoxy group, or a nitro group.

In the general formula (1) or (2), the protective group in the "protective group for a hydroxyl group for synthesis of nucleic acid" as R₁ and R₂, and in the "hydroxyl group protected with a protective group for synthesis of nucleic acid" as R₃, R₄ and R₅ and the α group is not limited, as long as the protective group can stably protect a hydroxyl group during synthesis of nucleic acid. Concretely, the protective group refers to a protective group which is stable under acidic or neutral conditions and which can be cleft by a chemical method such as hydrogenolysis, hydrolysis, electrolysis or photolysis. Examples of such a protective group are "aliphatic acyl groups", including alkylcarbonyl groups such as formyl, acetyl, propionyl, butyryl, isobutyryl, pentanoyl, pivaloyl, valeryl, isovaleryl, octanoyl, nonanoyl, decanoyl, 3-methylnonanoyl, 8-methylnonanoyl, 3-ethyloctanoyl, 3,7-dimethyloctanoyl, undecanoyl, dodecanoyl, tridecanoyl, tetradecanoyl, pentadecanoyl, hexadecanoyl, 1-methylpentadecanoyl, 14-methylpentadecanoyl, 13,13-dimethyltetradecanoyl, heptadecanoyl, 15-methylhexadecanoyl, octadecanoyl, 1-methylheptadecanoyl, nonadecanoyl, eicosanoyl, and heneicosanoyl, carboxylated alkylcarbonyl groups such as succinoyl, glutaroyl and adipoyl, halogeno lower alkylcarbonyl groups such as chloroacetyl, dichloroacetyl, trichloroacetyl and trifluoroacetyl, lower alkoxy lower alkylcarbonyl groups such as methoxyacetyl, and unsaturated alkylcarbonyl groups such as (E)-2-methyl-2-butenoyl; "lower alkyl groups" such as methyl, ethyl, n-propyl, isopropyl, n-butyl, isobutyl, s-butyl, tert-butyl, n-pentyl, isopentyl, 2-methylbutyl, neopentyl, 1-ethylpropyl, n-hexyl, isohexyl, 4-methylpentyl, 3-methylpentyl, 2-methylpentyl, 1-methylpentyl, 3,3-dimethylbutyl, 2,2-dimethylbutyl, 1,1-dimethylbutyl, 1,2-dimethylbutyl, 1,3-dimethylbutyl, 2,3-dimethylbutyl, and 2-ethylbutyl; "lower alkenyl groups" such as ethenyl, 1-propenyl, 2-propenyl, 1-methyl-2-propenyl, 1-methyl-1-propenyl, 2-methyl-1-propenyl, 2-methyl-2-propenyl, 2-ethyl-2-propenyl, 1-butenyl, 2-butenyl, 1-methyl-2-butenyl, 1-methyl-1-butenyl, 3-methyl-2-butenyl, 1-ethyl-2-butenyl, 3-butenyl, 1-methyl-3-butenyl, 2-methyl-3-butenyl, 1-ethyl-3-butenyl, 1-pentenyl, 2-pentenyl, 1-methyl-2-pentenyl, 2-methyl-2-pentenyl, 3-pentenyl, 1-methyl-3-pentenyl, 2-methyl-3-pentenyl, 4-pentenyl, 1-methyl-4-pentenyl, 2-methyl-4-pentenyl, 1-hexenyl, 2-hexenyl, 3-hexenyl, 4-hexenyl and 5-hexenyl; "aromatic acyl groups", including arylcarbonyl groups such as benzoyl, α-naphthoyl and β-naphthoyl, halogenoarylcarbonyl groups such as 2-bromobenzoyl and 4-chlorobenzoyl, lower alkylated arylcarbonyl groups such as 2,4,6-trimethylbenzoyl and 4-toluoyl, lower alkoxylated arylcarbonyl groups such as 4-anisoyl, carboxylated arylcarbonyl groups such as 2-carboxybenzoyl, 3-carboxybenzoyl and 4-carboxybenzoyl, nitrated arylcarbonyl groups such as 4-nitrobenzoyl and 2-nitrobenzoyl, lower alkoxycarbonylated arylcarbonyl groups such as 2-(methoxycarbonyl)benzoyl, and arylated arylcarbonyl groups such as 4-phenylbenzoyl;
"tetrahydropyranyl or tetrahydrothiopyranyl groups" such as tetrahydropyran-2-yl, 3-bromotetrahydropyran-2-yl. 4-methoxytetrahydropyran-4-yl, tetrahydrothiopyran-4-yl and 4-methoxytetrahydrothiopyran-4-yl; "tetrahydrofuranyl or tetrahydrothiofuranyl groups" such as tetrahydrofuran-2-yl and tetrahydrothiofuran-2-yl; "silyl groups", including tri-lower alkylsilyl groups such as trimethylsilyl, triethylsilyl, isopropyldimethylsilyl, t-butyldimethylsilyl, methyldiisopropylsilyl, methyldi-t-butylsilyl, and triisopropylsilyl, and tri-lower alkylsilyl groups substituted by 1 or 2 aryl groups, such as diphenylmethylsilyl, diphenylbutylsilyl, diphenylisopropylsilyl and phenyldiisopropylsilyl; "lower alkoxymethyl groups" such as methoxymethyl, 1,1-dimethyl-1-methoxymethyl, ethoxymethyl, propoxymethyl, isopropoxymethyl, butoxymethyl and t-butoxymethyl: "lower alkoxylated lower alkoxymethyl groups" such as 2-methoxyethoxymethyl; "halogeno lower alkoxymethyl groups" such as 2,2,2-trichloroethoxymethyl and bis(2-chloroethoxy)methyl; "lower alkoxylated ethyl groups" such as 1-ethoxyethyl and 1-(isopropoxy)ethyl; "halogenated ethyl groups" such as 2,2,2-trichloroethyl; "methyl groups substituted by 1 to 3 aryl groups", such as benzyl, α-naphthylmethyl, β-naphthylmethyl, diphenylmethyl, triphenylmethyl, α-naphthyldiphenylmethyl, and 9-anthrylmethyl; "methyl groups substituted by 1 to 3 aryl groups, with the aryl ring being substituted by a lower alkyl, lower alkoxy, halogen or cyano group", such as 4-methylbenzyl, 2,4,6-trimethylbenzyl, 3,4,5-trimethylbenzyl, 4-methoxybenzyl, 4-methoxyphenyldiphenylmethyl, 4,4'-dimethoxytriphenylmethyl, 2-nitrobenzyl, 4-nitrobenzyl, 4-chlorobenzyl, 4-bromobenzyl, and 4-cyanobenzyl; "lower alkoxycarbonyl groups" such as methoxycarbonyl, ethoxycarbonyl, t-butoxycarbonyl, and isobutoxycarbonyl; "aryl groups substituted by a halogen atom, a lower alkoxy group or a nitro group", such as 4-chlorophenyl, 2-fluorophenyl, 4-methoxyphenyl, 4-nitrophenyl, and 2,4-dinitrophenyl; "lower alkoxycarbonyl groups substituted by halogen or a tri-lower alkylsilyl group", such as 2,2,2-trichloroethoxycarbonyl and 2-trimethylsilylethoxycarbonyl; "alkenyloxycarbonyl groups" such as vinyloxycarbonyl and aryloxycarbonyl; and "aralkyloxycarbonyl groups having an aryl ring optionally substituted by 1 or 2 lower alkoxy or nitro groups", such as benzyloxycarbonyl, 4-methoxybenzyloxycarbonyl, 3,4-dimethoxybenzyloxycarbonyl, 2-nitrobenzyloxycarbonyl, and 4-nitrobenzyloxycarbonyl. In the "protective group for a hydroxyl group for synthesis of nucleic acid" as R₁ and R₂, the preferred protective group is the "aliphatic acyl group," "aromatic acyl group," "methyl group substituted by 1 to 3 aryl groups", "methyl group substituted by 1 to 3 aryl groups, with the aryl ring being substituted by a lower alkyl, lower alkoxy, halogen or cyano group", or "silyl group", the more preferred protective group being an acetyl group, a benzoyl group, a benzyl group, a p-methoxybenzoyl group, a dimethoxytrityl group, a monomethoxytrityl group, or a tert-butyldiphenylsilyl group. Preferred as the protective group in the "hydroxyl group protected with a protective group for synthesis of nucleic acid" as R₃, R₄ and R₅ or the α-group is the "aliphatic acyl group," "aromatic acyl group," "methyl group substituted by 1 to 3 aryl groups", "aryl group substituted by a halogen atom, a lower alkoxy group or a nitro group", "lower alkyl group" or "lower alkenyl group", the more preferred protective group being a benzoyl group, a benzyl group, a 2-chlorophenyl group, a 4-chlorophenyl group or a 2-propenyl group.

In the general formula (1) or (2), the "alkyl group" as R₁ and R₂ refers to a straight chain or branched chain alkyl group having 1 to 20 carbon atoms, including not only a straight chain or branched chain alkyl group having 1 to 6 carbon atoms (herein, also referred to as a lower alkyl group), such as methyl, ethyl, n-propyl, isopropyl, n-butyl, isobutyl, s-butyl, tert-butyl, n-pentyl, isopentyl, 2-methylbutyl, neopentyl, 1-ethylpropyl, n-hexyl, isohexyl, 4-methylpentyl, 3-methylpentyl, 2-methylpentyl, 1-methylpentyl, 3,3-dimethylbutyl, 2,2-dimethylbutyl, 1,1-dimethylbutyl, 1,2-dimethylbutyl, 1,3-dimethylbutyl, 2,3-dimethylbutyl, or 2-ethylbutyl, but also a straight chain or branched chain alkyl group having 7 to 20 carbon atoms, such as heptyl, octyl, nonyl or decyl. Preferred is the above-mentioned straight chain or branched chain alkyl group having 1 to 6 carbon atoms.

In the general formulas (1) or (2), the "alkenyl group" as R₁ and R₂ refers to a straight chain or branched chain alkenyl group having 2 to 20 carbon atoms, including not only a straight chain or branched chain alkenyl group having 2 to 6 carbon atoms (herein, also referred to as a lower alkenyl group), such as ethenyl, 1-propenyl, 2-propenyl, 1-methyl-2-propenyl, 1-methyl-l-propenyl, 2-methyl-1-propenyl, 2-methyl-2-propenyl, 2-ethyl-2-propenyl, 1-butenyl, 2-butenyl, 1-methyl-2-butenyl, 1-methyl-1-butenyl, 3-methyl-2-butenyl, 1-ethyl-2-butenyl, 3-butenyl, 1-methyl-3-butenyl, 2-methyl-3-butenyl, 1-ethyl-3-butenyl, 1-pentenyl, 2-pentenyl, 1-methyl-2-pentenyl, 2-methyl-2-pentenyl, 3-pentenyl, 1-methyl-3-pentenyl, 2-methyl-3-pentenyl, 4-pentenyl, 1-methyl-4-pentenyl, 2-methyl-4-pentenyl, 1-hexenyl, 2-hexenyl, 3-hexenyl, 4-hexenyl or 5-hexenyl, but also geranyl or farnesyl. Preferred is the above-mentioned straight chain or branched chain alkenyl group having 2 to 6 carbon atoms.

In the general formula (1) or (2), the "cycloalkyl group" as R₁ and R₂ refers to a cycloalkyl group having 3 to 10 carbon atoms, including, for example, cyclopropyl, cyclobutyl, cyclopentyl, cyclohexyl, cycloheptyl, cyclooctyl, norbornyl or adamantyl. Preferred is a cycloalkyl group having 3 to 8 carbon atoms, such as cyclopropyl, cyclobutyl, cyclopentyl, cyclohexyl, cycloheptyl or cyclooctyl. Also, the "cycloalkyl group" includes a heterocyclic group in which one or more methylene groups on the ring of the cycloalkyl group have been substituted by an oxygen atom, a sulfur atom or a nitrogen atom substituted by an alkyl group. An example of such a substituted heterocyclic group is a tetrahydropyranyl group.

In the general formula (1) or (2), the "aryl group" as R₁ and R₂ refers to a monovalent substituent having 6 to 14 carbon atoms, which is obtained by removing one hydrogen atom from an aromatic hydrocarbon group. Its examples are phenyl, indenyl, naphthyl, phenanthrenyl, and anthracenyl. Its aryl ring may be substituted by one or more of groups, including a halogen atom, a lower alkyl group, a hydroxyl group, an alkoxy group, an aryloxy group, an amino group, a nitro group, a trifluoromethyl group, and a phenyl group. Examples of the optionally substituted aryl group are 2-methylphenyl, 2,6-dimethylphenyl, 2-chlorophenyl, 4-chlorophenyl, 2,4-dichlorophenyl, 2,5-dichlorophenyl, 2-bromophenyl, 4-methoxyphenyl, 4-chloro-2-nitrophenyl, 4-nitrophenyl, 2,4-dinitrophenyl, and biphenyl. Preferred examples thereof are a phenyl group, and a phenyl group substituted by a halogen atom, a lower alkoxy group, or a nitro group.

In the general formulas (1) or (2), the "aralkyl group" as R₁ and R₂ refers to an alkyl group with 1 to 6 carbon atoms substituted by an aryl group. Examples of such an aryl-substituted alkyl group are "methyl groups substituted by 1 to 3 aryl groups", such as benzyl, α-naphthylmethyl, β-naphthylmethyl, indenylmethyl, phenanthrenylmethyl, anthracenylmethyl, diphenylmethyl, triphenylmethyl, α-naphthyldiphenylmethyl, and 9-anthrylmethyl: "methyl groups substituted by 1 to 3 aryl groups, with the aryl ring being substituted by a lower alkyl, lower alkoxy, halogen or cyano group", such as 4-methylbenzyl, 2,4,6-trimethylbenzyl, 3,4,5-trimethylbenzyl, 4-methoxybenzyl, 4-methoxyphenyldiphenylmethyl, 4,4'-dimethoxytriphenylmethyl, 2-nitrobenzyl, 4-nitrobenzyl, 4-chlorobenzyl, 4-bromobenzyl, and 4-cyanobenzyl; and "alkyl groups with 3 to 6 carbon atoms substituted by an aryl group", such as 1-phenethyl, 2-phenethyl, 1-naphthylethyl, 2-naphthylethyl, 1-phenylpropyl, 2-phenylpropyl, 3-phenylpropyl, 1-naphthylpropyl, 2-naphthylpropyl, 3-naphthylpropyl, 1-phenylbutyl, 2-phenylbutyl, 3-phenylbutyl, 4-phenylbutyl, 1-naphthylbutyl, 2-naphthylbutyl, 3-naphthylbutyl, 4-naphthylbutyl, 1-phenylpentyl, 2-phenylpentyl, 3-phenylpentyl, 4-phenylpentyl, 5-phenylpentyl, 1-naphthylpentyl, 2-naphthylpentyl, 3-naphthylpentyl, 4-naphthylpentyl, 5-naphthylpentyl, 1-phenylhexyl, 2-phenylhexyl, 3-phenylhexyl, 4-phenylhexyl, 5-phenylhexyl, 6-phenylhexyl, 1-naphthylpentyl, 2-naphthylpentyl, 3-naphthylpentyl, 4-naphthylpentyl, 5-naphthylpentyl, and 6-naphthylpentyl. Preferred are the "methyl groups substituted by 1 to 3 aryl groups" and "methyl groups substituted by 1 to 3 aryl groups, with the aryl ring being substituted by a lower alkyl, lower alkoxy, halogen or cyano group". More preferred are 4-methoxyphenyldiphenylmethyl and 4,4'-dimethoxytriphenylmethyl.

Examples of the "acyl group" as R₁ and R₂ in the general formula (1) or (2) are "aliphatic acyl groups", including alkylcarbonyl groups, such as formyl, acetyl, propionyl, butyryl, isobutyryl, pentanoyl, pivaloyl, valeryl, isovaleryl, octanoyl, nonanoyl, decanoyl, 3-methylnonanoyl, 8-methylnonanoyl, 3-ethyloctanoyl, 3,7-dimethyloctanoyl, undecanoyl, dodecanoyl, tridecanoyl, tetradecanoyl, pentadecanoyl, hexadecanoyl, 1-methylpentadecanoyl, 14-methylpentadecanoyl, 13,13-dimethyltetradecanoyl, heptadecanoyl, 15-methylhexadecanoyl, octadecanoyl, 1-methylheptadecanoyl, nonadecanoyl, eicosanoyl, and heneicosanoyl, carboxylated alkylcarbonyl groups such as succinoyl, glutaroyl and adipoyl, halogeno lower alkylcarbonyl groups such as chloroacetyl, dichloroacetyl, trichloroacetyl and trifluoroacetyl, lower alkoxy lower alkylcarbonyl groups such as methoxyacetyl, and unsaturated alkylcarbonyl groups such as (E)-2-methyl-2-butenoyl; and "aromatic acyl groups", including arylcarbonyl groups, such as benzoyl, α-naphthoyl and β-naphthoyl, halogenoarylcarbonyl groups such as 2-bromobenzoyl and 4-chlorobenzoyl, lower alkylated arylcarbonyl groups such as 2,4,6-trimethylbenzoyl and 4-toluoyl, lower alkoxylated arylcarbonyl groups such as 4-anisoyl, carboxylated arylcarbonyl groups such as 2-carboxybenzoyl, 3-carboxybenzoyl and 4-carboxybenzoyl, nitrated arylcarbonyl groups such as 4-nitrobenzoyl and 2-nitrobenzoyl, lower alkoxycarbonylated arylcarbonyl groups such as 2-(methoxycarbonyl)benzoyl, and arylated arylcarbonyl groups such as 4-phenylbenzoyl. Preferred examples are formyl, acetyl, propionyl, butyryl, isobutyryl, pentanoyl, pivaloyl, and benzoyl groups.

Examples of the "silyl group" as R₁ and R₂ in the general formula (1) or (2) are "tri-lower alkylsilyl groups" such as trimethylsilyl, triethylsilyl, isopropyldimethylsilyl, t-butyldimethylsilyl, methyldiisopropylsilyl, methyldi-t-butylsilyl, and triisopropylsilyl, and "tri-lower alkylsilyl groups substituted by 1 or 2 aryl groups", such as diphenylmethylsilyl, butyldiphenylbutylsilyl, diphenylisopropylsilyl and phenyldiisopropylsilyl. Preferred examples are trimethylsilyl, triethylsilyl, triisopropylsilyl, t-butyldimethylsilyl, and t-butyldiphenylsilyl. A more preferred example is trimethylsilyl.

In the general formula (1) or (2), the "protective group" in the "phosphate group protected with a protective group for synthesis of nucleic acid" as R₁ and R₂ is not limited, as long as the protective group can stably protect a phosphate group during synthesis of nucleic acid. Concretely, the protective group refers to a protective group which is stable under acidic or neutral conditions and which can be cleft by a chemical method such as hydrogenolysis, hydrolysis, electrolysis or photolysis. Examples of such a protective group are "lower alkyl groups" such as methyl, ethyl, n-propyl, isopropyl, n-butyl, isobutyl, s-butyl, tert-butyl, n-pentyl, isopentyl, 2-methylbutyl, neopentyl, 1-ethylpropyl, n-hexyl, isohexyl, 4-methylpentyl, 3-methylpentyl, 2-methylpentyl, 1-methylpentyl, 3,3-dimethylbutyl, 2,2-dimethylbutyl, 1,1-dimethylbutyl, 1,2-dimethylbutyl, 1,3-dimethylbutyl, 2,3-dimethylbutyl, and 2-ethylbutyl; "cyanated lower alkyl groups" such as 2-cyanoethyl, and 2-cyano-1,1-dimethylethyl; "ethyl groups substituted by a silyl group", such as 2-methyldiphenylsilylethyl, 2-trimethylsilylethyl and 2-triphenylsilylethyl; "halogenated lower alkyl groups" such as 2,2,2-trichloroethyl, 2,2,2-tribromoethyl, 2,2,2-trifluoroethyl and 2,2,2-trichloro-1,1-dimethylethyl; "lower alkenyl groups" such as ethenyl, 1-propenyl, 2-propenyl, 1-methyl-2-propenyl, 1-methyl-1-propenyl, 2-methyl-2-propenyl, 2-ethyl-2-propenyl, 1-butenyl, 2-butenyl, 1-methyl-2-butenyl, 1-methyl-1-butenyl, 3-methyl-2-butenyl, 1-ethyl-2-butenyl, 3-butenyl, 1-methyl-3-butenyl, 2-methyl-3-butenyl, 1-ethyl-3-butenyl, 1-pentenyl, 2-pentenyl, 1-methyl-2-pentenyl, 2-methyl-2-pentenyl, 3-pentenyl, 1-methyl-3-pentenyl, 2-methyl-3-pentenyl, 4-pentenyl, 1-methyl-4-pentenyl, 2-methyl-4-pentenyl, 1-hexenyl, 2-hexenyl, 3-hexenyl, 4-hexenyl and 5-hexenyl; "cycloalkyl groups" such as cyclopropyl, cyclobutyl, cyclopentyl, cyclohexyl, cycloheptyl, norbornyl or adamantyl; "cyanated lower alkenyl groups" such as 2-cyanobutenyl; "aralkyl groups" such as benzyl, α-naphthylmethyl, β-naphthylmethyl, indenylmethyl, phenanthrenylmethyl, anthracenylmethyl, diphenylmethyl, triphenylmethyl, 1-phenethyl, 2-phenethyl, 1-naphthylethyl, 2-naphthylethyl, 1-phenylpropyl, 2-phenylpropyl, 3-phenylpropyl, 1-naphthylpropyl, 2-naphthylpropyl, 3-naphthylpropyl, 1-phenylbutyl, 2-phenylbutyl, 3-phenylbutyl, 4-phenylbutyl, 1-naphthylbutyl, 2-naphthylbutyl, 3-naphthylbutyl, 4-naphthylbutyl, 1-phenylpentyl, 2-phenylpentyl, 3-phenylpentyl, 4-phenylpentyl, 5-phenylpentyl, 1-naphthylpentyl, 2-naphthylpentyl, 3-naphthylpentyl, 4-naphthylpentyl, 5-naphthylpentyl, 1-phenylhexyl, 2-phenylhexyl, 3-phenylhexyl, 4-phenylhexyl, 5-phenylhexyl, 6-phenylhexyl, 1-naphthylpentyl, 2-naphthylpentyl, 3-naphthylpentyl. 4-naphthylpentyl, 5-naphthylpentyl, and 6-naphthylpentyl; "aralkyl groups having an aryl ring substituted by a nitro group or a halogen atom", such as 4-chlorobenzyl, 2-(4-nitrophenyl)ethyl, o-nitrobenzyl, 4-nitrobenzyl, 2,4-dinitrobenzyl, and 4-chloro-2-nitrobenzyl; "aryl groups" such as phenyl, indenyl, naphthyl, phenanthrenyl and anthracenyl; and "aryl groups substituted by a lower alkyl group, a halogen atom or a nitro group", such as 2-methylphenyl, 2,6-dimethylphenyl, 2-chlorophenyl, 4-chlorophenyl, 2,4-dichlorophenyl, 2,5-dichlorophenyl, 2-bromophenyl, 4-nitrophenyl, and 4-chloro-2-nitrophenyl. Preferred examples are the "lower alkyl groups", "lower alkyl groups substituted by a cyano group", "aralkyl groups", "aralkyl groups having an aryl ring substituted by a nitro group or a halogen atom", or "aryl groups substituted by a lower alkyl group, a halogen atom or a nitro group". A more preferred examples is a 2-cyanoethyl group, a 2,2,2-trichloroethyl group, a benzyl group, a 2-chlorophenyl group, or a 4-chlorophenyl group.

In the general formula (1) or (2), the protective group in the "mercapto group protected with a protective group for synthesis of nucleic acid" as R₃, R₄ and R₅ and the α group is not limited, as long as the protective group can stably protect a mercapto group during synthesis of nucleic acid. Concretely, the protective group refers to a protective group which is stable under acidic or neutral conditions and which can be cleft by a chemical method such as hydrogenolysis, hydrolysis, electrolysis or photolysis. Examples of such a protective group are not only those named above as the protective group for a hydroxyl group, but also "disulfide-forming groups", including alkylthio groups such as methylthio, ethylthio and tert-butylthio, and arylthio groups such as benzylthio. Preferred examples are "aliphatic acyl groups" or "aromatic acyl groups". More preferred examples are a benzoyl group and a benzyl group.

Examples of the "alkoxy groups having 1 to 5 carbon atoms" as R₃, R₄ and R₅ as well as the α group in the general formula (1) or (2) are methoxy, ethoxy, n-propoxy, isopropoxy, n-butoxy, isobutoxy, s-butoxy, tert-butoxy, and n-pentoxy. A preferred example is a methoxy or ethoxy group.

Examples of the "alkoxythio groups having 1 to 5 carbon atoms" as R₃, R₄ and R₅ as well as the α group in the general formula (1) or (2) are methylthio, ethylthio, propylthio, isopropylthio, butylthio, isobutylthio, s-butylthio, tert-butylthio, and n-pentylthio. A preferred example is a methylthio or ethylthio group.

The "cyanoalkoxy group having 1 to 6 carbon atoms" as R₃, R₄ and R₅ in the general formula (1) or (2) refers to the above-mentioned "alkoxy group having 1 to 5 carbon atoms" which has been substituted by a cyano group. Examples of such a cyano-substituted alkoxy group are cyanomethoxy, 2-cyanoethoxy, 3-cyanopropoxy, 4-cyanobutoxy, 3-cyano-2-methylpropoxy, and 1-cyanomethyl-1,1-dimethylmethoxy. A preferred example is a 2-cyanoethoxy group.

Examples of the "amino group substituted by an alkyl group having 1 to 5 carbon atoms" as R₃, R₄ and R₅ as well as the α group in the general formula (1) or (2) are methylamino, ethylamino, propylamino, isopropylamino, butylamino, isobutylamino, s-butylamino, tert-butylamino, dimethylamino, diethylamino, dipropylamino, diisopropylamino, dibutylamino, diisobutylamino, di(s-butyl)amino, and di(tert-butyl)amino. A preferred example is a methylamino, ethylamino, dimethylamino, diethylamino or diisopropylamino group.

Examples of the "alkyl group having 1 to 5 carbon atoms" as the α group are methyl, ethyl, propyl, isopropyl, isopropyl, butyl, isobutyl, s-butyl, tert-butyl, and n-pentyl. A preferred example is a methyl or ethyl group.

As the "halogen atom" defining R₃ and the α group, a fluorine atom, a chlorine atom, a bromine atom or an iodine atom, for example, can be named. A preferred example thereof is a fluorine atom or a chlorine atom.

The "phosphoroamidite group" refers to a group represented by the formula -P(OR₁ₐ)(NR_{1b}) (where R₁ₐ represents an alkyl group having 1 or more carbon atoms or a cyanoalkyl group having 1 to 7 carbon atoms, and R_{1b} represents an alkyl group having 1 to 6 carbon atoms). Its preferred example is a group represented by the formula - P(OC₂H₄CN)(N(iPr)₂) or a group represented by the formula -P(OCH₃)(N(iPr)₂).

The protective group in the "amino group protected with a protective group for synthesis of nucleic acid" as R₃ and the α group is not limited, as long as the protective group can stably protect an amino group during synthesis of nucleic acid. Concretely, the protective group refers to a protective group which is stable under acidic or neutral conditions and which can be cleft by a chemical method such as hydrogenolysis, hydrolysis, electrolysis or photolysis. Examples of such a protective group are "aliphatic acyl groups", including alkylcarbonyl groups, such as formyl, acetyl, propionyl, butyryl, isobutyryl, pentanoyl, pivaloyl, valeryl, isovaleryl, octanoyl, nonanoyl, decanoyl, 3-methylnonanoyl, 8-methylnonanoyl, 3-ethyloctanoyl, 3,7-dimethyloctanoyl, undecanoyl, dodecanoyl, tridecanoyl, tetradecanoyl, pentadecanoyl, hexadecanoyl, 1-methylpentadecanoyl, 14-methylpentadecanoyl, 13,13-dimethyltetradecanoyl, heptadecanoyl, 15-methylhexadecanoyl, octadecanoyl, 1-methylheptadecanoyl, nonadecanoyl, nonadecanoyl, eicosanoyl, and heneicosanoyl, carboxylated alkylcarbonyl groups such as succinoyl, glutaroyl and adipoyl, halogeno lower alkylcarbonyl groups such as chloroacetyl, dichloroacetyl, trichloroacetyl and trifluoroacetyl, lower alkoxy lower alkylcarbonyl groups such as methoxyacetyl, and unsaturated alkylcarbonyl groups such as (E)-2-methyl-2-butenoyl; "aromatic acyl groups", including arylcarbonyl groups, such as benzoyl, α-naphthoyl and β-naphthoyl, halogenoarylcarbonyl groups such as 2-bromobenzoyl and 4-chlorobenzoyl, lower alkylated arylcarbonyl groups such as 2,4,6-trimethylbenzoyl and 4-toluoyl, lower alkoxylated arylcarbonyl groups such as 4-anisoyl, carboxylated arylcarbonyl groups such as 2-carboxybenzoyl, 3-carboxybenzoyl and 4-carboxybenzoyl, nitrated arylcarbonyl groups such as 4-nitrobenzoyl and 2-nitrobenzoyl, lower alkoxycarbonylated arylcarbonyl groups such as 2-(methoxycarbonyl)benzoyl, and arylated arylcarbonyl groups such as 4-phenylbenzoyl; "lower alkoxycarbonyl groups" such as methoxycarbonyl, ethoxycarbonyl, t-butoxycarbonyl, and isobutoxycarbonyl; "lower alkoxycarbonyl groups substituted by halogen or a tri-lower alkylsilyl group", such as 2,2,2-trichloroethoxycarbonyl and 2-trimethylsilylethoxycarbonyl; "alkenyloxycarbonyl groups" such as vinyloxycarbonyl and aryloxycarbonyl; and "aralkyloxycarbonyl groups having an aryl ring optionally substituted by one or two lower alkoxy or nitro groups", such as benzyloxycarbonyl, 4-methoxybenzyloxycarbonyl, 2-nitrobenzyloxycarbonyl, and 4-nitrobenzyloxycarbonyl. Preferred examples of the protective group are the "aliphatic acyl groups" or "aromatic acyl groups", and a more preferred example is a benzoyl group.

The term "nucleoside analogues" refers to non-natural type nucleosides among "nucleosides" comprising purine or pyrimidine bases and sugars linked together, and also those comprising aromatic heterocyclic rings or aromatic hydrocarbon rings, which are other than purine and pyrimidine and which can be used instead of purine and pyrimidine, and sugars linked thereto.

The "oligonucleotide analogues" refer to non-natural type derivatives of "oligonucleotides" consisting of 2 to 50 identical or different "nucleosides" or "nucleoside analogues" linked together by phosphodiester bonds. Preferred examples of such analogues are sugar derivatives with the sugar portion modified; thioate derivatives with the phosphodiester portion thioated; ester compounds with the terminal phosphate portion esterified; and amide compounds with the amino group on the purine base being amidated. More preferred examples are the sugar derivatives with the sugar portion modified.

The "salts thereof" refer to salts of the compounds (1) of the present invention, because these compounds can be converted into salts. Preferred examples of such salts are metal salts including alkali metal salts such as sodium salts, potassium salts and lithium salts, alkaline earth metal salts such as calcium salts and magnesium salts, and aluminum salts, iron salts, zinc salts, copper salts, nickel salts and cobalt salts; amine salts, including inorganic salts such as ammonium salts, and organic salts such as t-octylamine salts, dibenzylamine salts, morpholine salts, glucosamine salts, phenylglycine alkyl ester salts, ethylenediamine salts, N-methylglucamine salts, guanidine salts, diethylamine salts, triethylamine salts, dicyclohexylamine salts, N,N'-dibenzylethylenediamine salts, chloroprocaine salts, procaine salts, diethanolamine salts, N-benzyl-phenethylamine salts, piperazine salts, tetramethylammonium salts, and tris(hydroxymethyl)aminomethane salts; inorganic acid salts, including halogenated hydroacid salts such as hydrofluorides, hydrochlorides, hydrobromides and hydriodides, nitrates, perchlorates, sulfates and phosphates; organic acid salts, including lower alkane sulfonic acid salts such as methanesulfonates, trifluoromethanesulfonates, and ethanesulfonates, aryl sulfonic acid salts such as benzenesulfonates and p-toluenesulfonates, acetates, malates, fumarates, succinates, citrates, tartarates, oxalates, and maleates; and amino acid salts, such as glycine salts, lysine salts, arginine salts, ornithine salts, glutamates and aspartates.

The "pharmacologically acceptable salts thereof" refer to salts of the oligonucleotide analogues of the present invention, because these analogues can be converted into salts. Preferred examples of such salts are metal salts including alkali metal salts such as sodium salts, potassium salts and lithium salts, alkaline earth metal salts such as calcium salts and magnesium salts, and aluminum salts, iron salts, zinc salts, copper salts, nickel salts and cobalt salts; amine salts, including inorganic salts such as ammonium salts, and organic salts such as t-octylamine salts, dibenzylamine salts, morpholine salts, glucosamine salts, phenylglycine alkyl ester salts, ethylenediamine salts, N-methylglucamine salts, guanidine salts, diethylamine salts, triethylamine salts, dicyclohexylamine salts, N,N'-dibenzylethylenediamine salts, chloroprocaine salts, procaine salts, diethanolamine salts, N-benzyl-phenethylamine salts, piperazine salts, tetramethylammonium salts, and tris(hydroxymethyl)aminomethane salts; inorganic acid salts, including halogenated hydroacid salts such as hydrofluorides, hydrochlorides, hydrobromides and hydriodides, nitrates, perchlorates, sulfates and phosphates; organic acid salts, including lower alkane sulfonic acid salts such as methanesulfonates, trifluoromethanesulfonates, and ethanesulfonates, aryl sulfonic acid salts such as benzenesulfonates and p-toluenesulfonates, acetates, malates, fumarates, succinates, citrates, tartarates, oxalates, and maleates; and amino acid salts, such as glycine salts, lysine salts, arginine salts, ornithine salts, glutamates and aspartates.

Of the compounds (1) and salts thereof according to the present invention, the preferred compounds are, for example, as follows:
(1) Compounds and salts thereof, wherein R₁ represents a hydrogen atom, an aliphatic acyl group, an aromatic acyl group, a methyl group substituted by 1 to 3 aryl groups, a methyl group substituted by 1 to 3 aryl groups, with the aryl rings being substituted by a lower alkyl, lower alkoxy, halogen or cyano group, or a silyl group.
(2) Compounds and salts thereof, wherein R₁ represents a hydrogen atom, an acetyl group, a benzoyl group, a benzyl group, a p-methoxybenzyl group, a dimethoxytrityl group, a monomethoxytrityl group, or a tert-butyldiphenylsilyl group.
(3) Compounds and salts thereof, wherein R₂ represents a hydrogen atom, an aliphatic acyl group, an aromatic acyl group, a methyl group substituted by 1 to 3 aryl groups, a methyl group substituted by 1 to 3 aryl groups, with the aryl rings being substituted by a lower alkyl, lower alkoxy, halogen or cyano group, a silyl group, a phosphoroamidite group, a phosphonyl group, a phosphate group, or a phosphate group protected with a protective group for synthesis of nucleic acid.
(4) Compounds and salts thereof, wherein R₂ represents a hydrogen atom, an acetyl group, a benzoyl group, a benzyl group, a p-methoxybenzyl group, a tert-butyldiphenylsilyl group, -P(OC₂H₄CN)(N(iPr)₂), -P(OCH₃) (N(iPr)₂) , a phosphonyl group, or a 2-chlorophenyl- or 4-chlorophenylphosphate group.
(5) Compounds and salts thereof, wherein R₃ represents a hydrogen atom, a halogen atom, a hydroxyl group, a hydroxyl group protected with a protective group for synthesis of nucleic acid, or an alkoxy group having 1 to 5 carbon atoms.
(6) Compounds and salts thereof, wherein A represents methylene.
(7) Compounds and salts thereof, wherein B represents a 6-aminopurin-9-yl (i.e. adeninyl) group, a 6-aminopurin-9-yl group having an amino group protected with a protective group for synthesis of nucleic acid, a 2,6-diaminopurin-9-yl group, a 2-amino-6-chloropurin-9-yl group, a 2-amino-6-chloropurin-9-yl group having an amino group protected with a protective group for synthesis of nucleic acid, a 2-amino-6-fluoropurin-9-yl group, a 2-amino-6-fluoropurin-9-yl group having an amino group protected with a protective group for synthesis of nucleic acid, a 2-amino-6-bromopurin-9-yl group, a 2-amino-6-bromopurin-9-yl group having an amino group protected with a protective group for synthesis of nucleic acid, a 2-amino-6-hydroxypurin-9-yl (i.e. guaninyl) group, a 2-amino-6-hydroxypurin-9-yl group having an amino group protected with a protective group for synthesis of nucleic acid, a 6-amino-2-methoxypurin-9-yl group, a 6-amino-2-chloropurin-9-yl group, a 6-amino-2-fluoropurin-9-yl group, a 2,6-dimethoxypurin-9-yl group, a 2,6-dichloropurin-9-yl group, a 6-mercaptopurin-9-yl group, a 2-oxo-4-amino-1,2-dihydropyrimidin-1-yl (i.e. cytosinyl) group, a 2-oxo-4-amino-1,2-dihydropyrimidin-1-yl group having an amino group protected with a protective group for synthesis of nucleic acid, a 2-oxo-4-amino-5-fluoro-1,2-dihydropyrimidin-1-yl group, a 2-oxo-4-amino-5-fluoro-1,2-dihydropyrimidin-1-yl group having an amino group protected with a protective group for synthesis of nucleic acid, a 4-amino-2-oxo-5-chloro-1,2-dihydropyrimidin-1-yl group, a 2-oxo-4-methoxy-1,2-dihydropyrimidin-1-yl group, a 2-oxo-4-mercapto-1,2-dihydropyrimidin-1-yl group, a 2-oxo-4-hydroxy-1,2-dihydropyrimidin-1-yl (i.e. uracinyl) group, a 2-oxo-4-hydroxy-5-methyl-1,2-dihydropyrimidin-1-yl (i.e. thyminyl) group, a 4-amino-5-methyl-2-oxo-1,2-dihydropyrimidin-1-yl (i.e. 5-methylcytosinyl) group, or a 4-amino-5-methyl-2-oxo-1,2-dihydropyrimidin-1-yl group having an amino group protected with a protective group for synthesis of nucleic acid.
(8) Compounds and salts thereof, wherein B represents a benzoylaminopurin-9-yl, adenyl, 2-isobutyrylamino-6-hydroxypurin-9-yl, guaninyl, 2-oxo-4-benzoylamino-1,2-dihydropyrimidin-1-yl, cytosinyl, 2-oxo-5-methyl-4-benzoylamino-1,2-dihydropyrimidin-1-yl, 5-methylcytosinyl, uracinyl or thyminyl group.
   In connection with the above compounds (1) to (2), (3) to (4) or (7) to (8), the greater their number is, the more preferred these compounds become. Also preferred are compounds and salts thereof obtained by arbitrarily selecting, in the general formula (1), R₁ from the compounds and salts (1) to (2), R₂ from the compounds and salts (3) to (4), R₃ from the compounds and salts (5), A from the compounds and salts (6), and B from the compounds and salts (7) to (8), or by arbitrarily combining these selections. Particularly preferred combinations are (2)-(3)-(5)-(6)-(7), (2)-(3)-(5)-(6)-(8), (2)-(4)-(5)-(6)-(7) and (2)-(4)-(5)-(6)-(8).
   Of the compounds of the general formula (1) and salts thereof, particularly preferred are the compounds and salts thereof selected from the following groups: 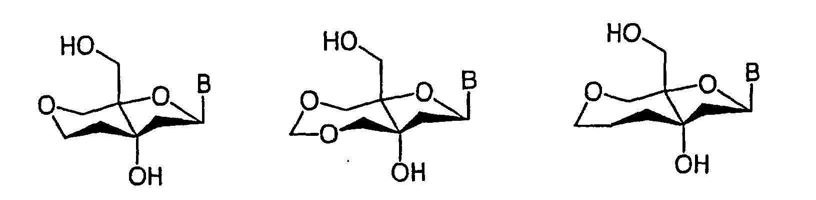
   In the structural formulas of the above groups, B is as defined earlier.
   Of oligonucleotide analogues containing one or more units having the structure represented by the general formula (2) according to the present invention, or pharmacologically acceptable salts thereof, the preferred ones are, for example, as follows:
(9) Oligonucleotide analogues and pharmacologically acceptable salts thereof, wherein A represents a methylene group).
(10) Oligonucleotide analogues and pharmacologically acceptable salts thereof, wherein R₃ represents a hydrogen atom, a halogen atom, a hydroxyl group, a hydroxyl group protected with a protective group for synthesis of nucleic acid, or an alkoxy group having 1 to 5 carbon atoms.
(11) Oligonucleotide analogues and pharmacologically acceptable salts thereof, wherein B represents a 6-aminopurin-9-yl (i.e. adeninyl) group, a 6-aminopurin-9-yl group having an amino group protected with a protective group for synthesis of nucleic acid, a 2,6-diaminopurin-9-yl group, a 2-amino-6-chloropurin-9-yl group, a 2-amino-6-chloropurin-9-yl group having an amino group protected with a protective group for synthesis of nucleic acid, a 2-amino-6-fluoropurin-9-yl group, a 2-amino-6-fluoropurin-9-yl group having an amino group protected with a protective group for synthesis of nucleic acid, a 2-amino-6-bromopurin-9-yl group, a 2-amino-6-bromopurin-9-yl group having an amino group protected with a protective group for synthesis of nucleic acid, a 2-amino-6-hydroxypurin-9-yl (i.e. guaninyl) group, a 2-amino-6-hydroxypurin-9-yl group having an amino group protected with a protective group for synthesis of nucleic acid, a 6-amino-2-methoxypurin-9-yl group, a 6-amino-2-chloropurin-9-yl group, a 6-amino-2-fluoropurin-9-yl group, a 2,6-dimethoxypurin-9-yl group, a 2,6-dichloropurin-9-yl group, a 6-mercaptopurin-9-yl group, a 2-oxo-4-amino-1,2-dihydropyrimidin-1-yl (i.e. cytosinyl) group, a 2-oxo-4-amino-1,2-dihydropyrimidin-1-yl group having an amino group protected with a protective group for synthesis of nucleic acid, a 2-oxo-4-amino-5-fluoro-1,2-dihydropyrimidin-1-yl group, a 2-oxo-4-amino-5-fluoro-1,2-dihydropyrimidin-1-yl group having an amino group protected with a protective group for synthesis of nucleic acid, a 4-amino-2-oxo-5-chloro-1,2-dihydropyrimidin-1-yl group, a 2-oxo-4-methoxy-1,2-dihydropyrimidin-1-yl group, a 2-oxo-4-mercapto-1,2-dihydropyrimidin-1-yl group, a 2-oxo-4-hydroxy-1,2-dihydropyrimidin-1-yl (i.e. uracinyl) group, a 2-oxo-4-hydroxy-5-methyl-1,2-dihydropyrimidin-1-yl (i.e. thyminyl) group, a 4-amino-5-methyl-2-oxo-l,z-dihydropyrimidin-1-yl (i.e. 5-methylcytosinyl) group, or a 4-amino-5-methyl-2-oxo-1,2-dihydropyrimidin-1-yl group having an amino group protected with a protective group for synthesis of nucleic acid.
(12) Oligonucleotide analogues and pharmacologically acceptable salts thereof, wherein B represents a benzoylaminopurin-9-yl, adenyl, 2-isobutyrylamino-6-hydroxypurin-9-yl, guaninyl, 2-oxo-4-benzoylamino-1,2-dihydropyrimidin-1-yl, cytosinyl, 2-oxo-5-methyl-4-benzoylamino-1,2-dihydropyrimidin-1-yl, 5-methylcytosinyl, uracinyl or thyminyl group.

In connection with the above oligonucleotide analogues (11) to (12), the greater their number is, the more preferred these oligonucleotide analogues become. Also preferred are oligonucleotide analogues and pharmacologically acceptable salts thereof obtained by arbitrarily selecting A from (9), R₃ from (10), and B from (11) to (12), or by arbitrarily combining these structures. Particularly preferred combinations for the general formula (2) are (9)-(10)-(11) and (9)-(10)-(12).

The nucleoside analogues and oligonucleotide analogues of the present invention can be synthesized based on the methods described in the Examples and the conventional technologies in the art.

### (1) Synthesis of nucleoside analogues

The compounds represented by the general formula (1) can be synthesized based on the methods described in the Examples and the conventional technologies in the art. The reaction conditions, protective group introducing reagents, and reaction reagents can be determined concretely by reference to the methods described in the Examples, but are not limited thereto. The reaction conditions and reagents usable based on common knowledge in the art can be adopted where appropriate. For example, the methods described in Japanese Patent Application Laid-Open No. 2000-297097 and Japanese Patent Application Laid-Open No. 1998-304889 can be referred to. Moreover, if B in the general formula (1) or (2) is any of various natural or non-natural nucleic acid bases and other aromatic heterocyclic rings or aromatic hydrocarbon rings, the starting material for the compounds of the present invention can be synthesized by referring to the method described in Japanese Patent Application Laid-Open No. 1998-304889.

### (2) Synthesis of oligonucleotide analogues

Various oligonucleotide analogues containing the nucleoside analogues of the present invention can be synthesized with the use of a publicly known DNA synthesizer. Then, the resulting oligonucleotide analogues are purified by use of a reversed phase column, and the purity of the products is analyzed by reversed phase HPLC, whereby the production of purified oligonucleotide analogues can be confirmed.

One or more of the nucleoside analogues of the present invention can be rendered existent in the oligonucleotide analogues. Alternatively, they may be present at 2 or more positions of the oligonucleotide analogue so as to be separated by one or more natural nucleotides. According to the present invention, it is possible to synthesize oligonucleotide analogues having the nucleotide analogues of the present invention introduced at the necessary positions and in the necessary numbers (over the necessary length). The entire length of the nucleotide analogues is 2 to 50, preferably 8 to 30, nucleotide units.

The nucleotide analogues of the present invention are minimally degraded by nucleases, and can exist for long periods in vivo after administration into living organisms. These nucleotide analogues form double strands with sense RNA, for example, to inhibit transcription into mRNA for formation of a pathogenic in vivo component (protein). The nucleotide analogues are also considered to inhibit the proliferation of infecting virus. In view of these facts, the nucleotide analogues of the present invention are expected to be useful as pharmaceuticals, including antitumor agents and antiviral agents, which inhibit the action of genes to treat diseases. That is, according to the present invention, there are provided oligonucleotide analogues, which have stable and excellent antisense or antigene activity or excellent activity as drugs for detection of particular genes or as primers for initiation of amplification of particular genes, and nucleoside analogues which are intermediates for production of the oligonucleotide analogues.

The nucleoside analogues of the present invention are subjected to trans-bridging between the 3-position and the 4-position thereof, whereby the conformation of the sugar portion can be fixed in S-form. As a result, oligonucleotide analogues sterically very similar to DNA molecules can be provided. Thus, sequence-specific potent binding affinity for single-stranded DNA, high nuclease activity, fixing in B-form in double-stranded DNA structures, and stabilization of triple-stranded nucleic acids are named as the particularly expected functions of the oligonucleotide analogues of the present invention.

Hence, the oligonucleotide analogues of the present invention are useful as materials for various physiologically active or bioactive substances and pharmaceuticals, as functional materials for RNAi and decoy double-stranded oligonucleotides, as functional materials for DNA chips and molecular beacons targeting single-stranded nucleic acids such as cDNA, as functional materials for uses in various antisense methods (including ribozymes and DNAzymes) or antigene methods, and as materials for high sensitivity analysis of in vivo trace components by combination with fluorescent or luminescent substances.

The nucleotide analogues of the present invention can be formed, for example, into parenteral preparations when mixed with customary adjuvants such as buffers and/or stabilizers. For topical use, they can be mixed with customary pharmaceutical carriers to prepare ointments, creams, liquids and solutions, or plasters.

The nucleoside analogues and oligonucleotide analogues of the present invention were synthesized in accordance with the synthesis scheme described below. Their syntheses will be described in further detail by the Examples.

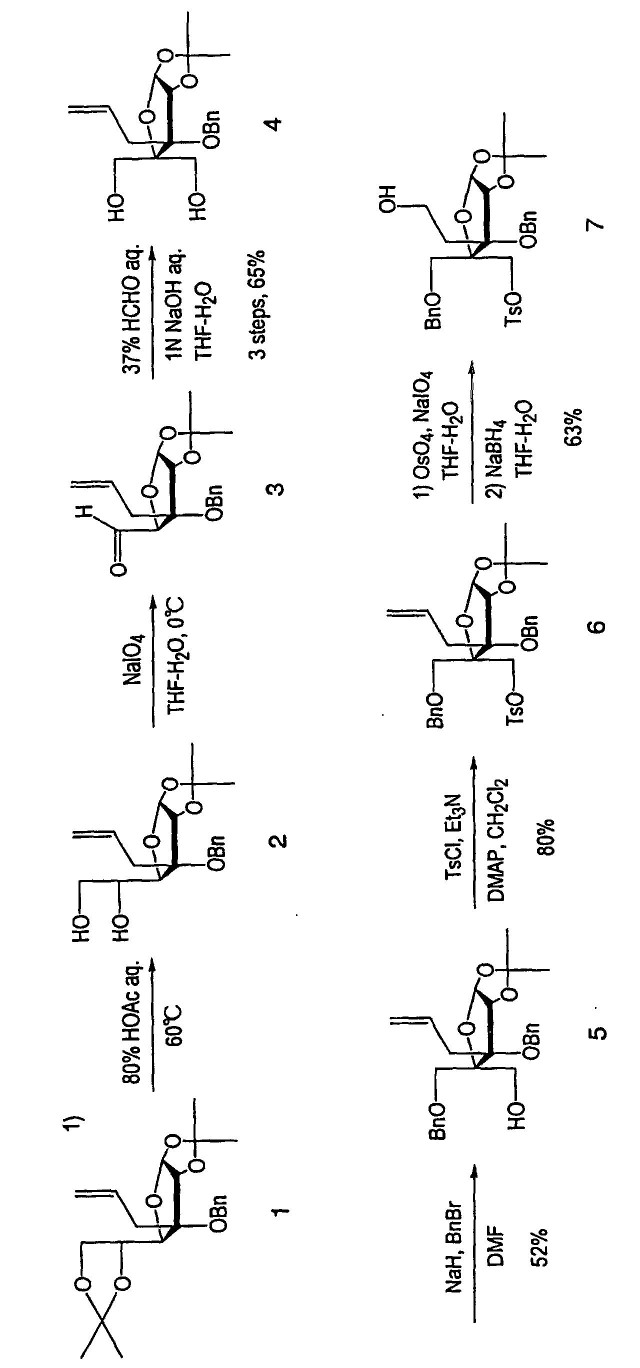

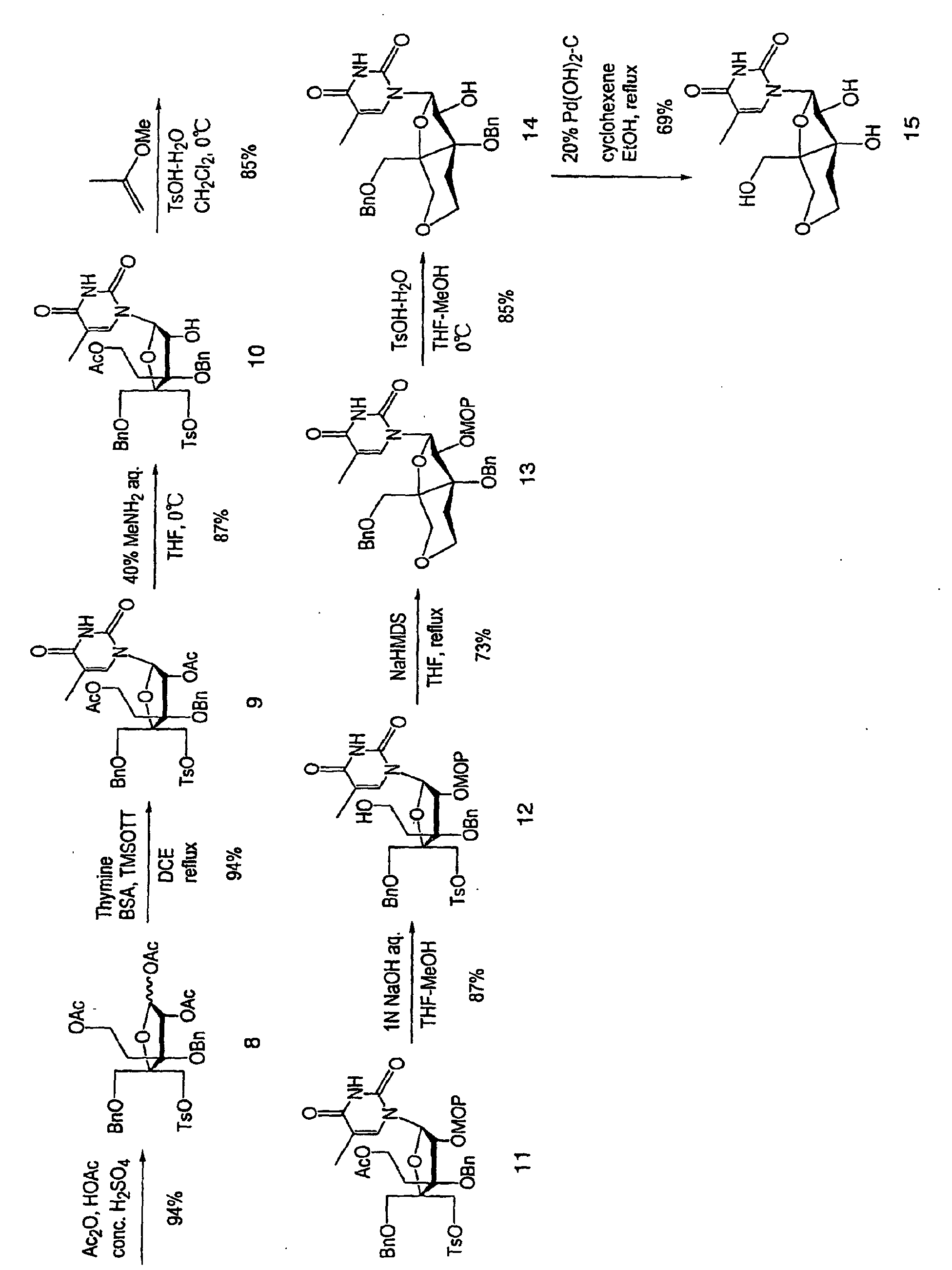

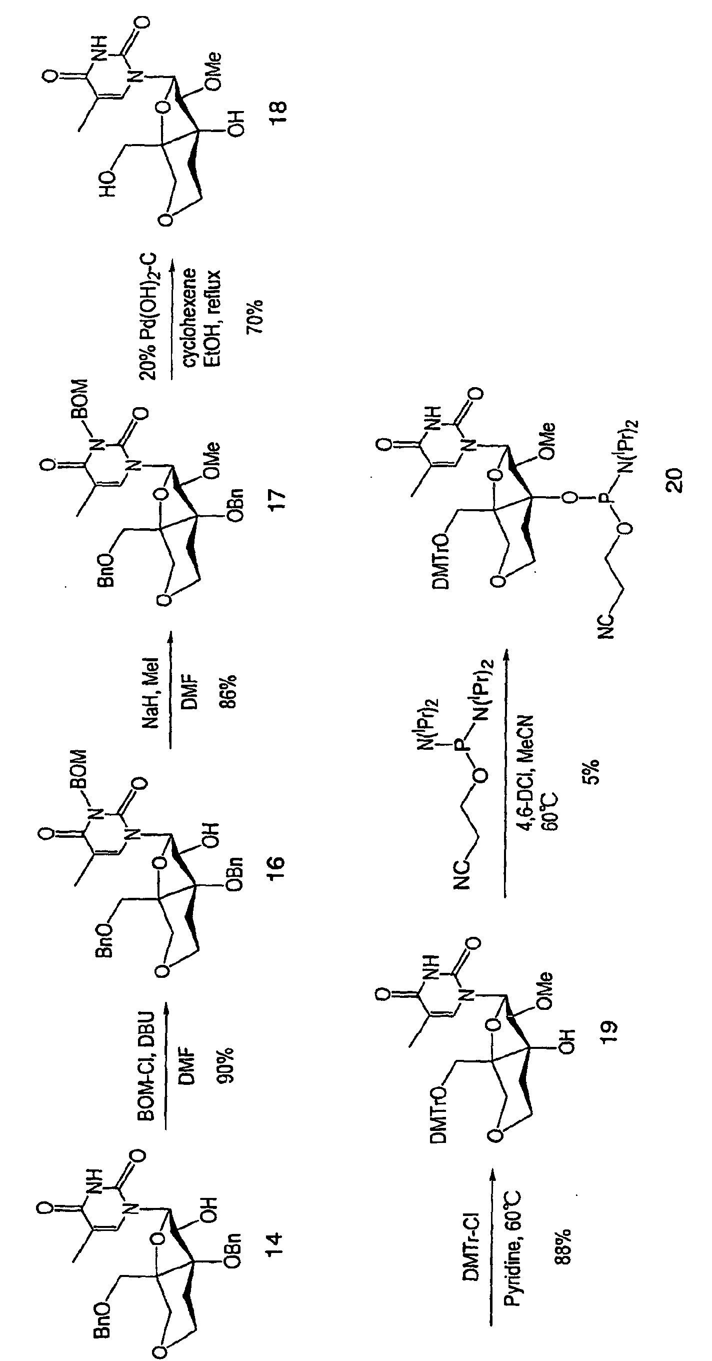

### [Example 1] Synthesis of nucleoside analogue:

### Synthesis of 5-methyl-3'-C,4'-C-ethoxymethyleneuridine (Compound 15)

### (1) 3-C-Allyl-3-0-benzyl-4-C-hydroxymethyl-1,2-0-isopropylidene-α-D-ribofuranose (Compound 4)

An 80% aqueous acetic acid solution (85 ml) of Compound 1 (4.9 g, 0.013 mol) was stirred for 3 hours at 40°C. After neutralization with a 10 N aqueous solution of sodium hydroxide, the mixture was extracted with ethyl acetate. The organic layer was washed with a saturated aqueous solution of sodium bicarbonate, water, and a saturated aqueous solution of sodium chloride, and then dried over anhydrous sodium sulfate. The solvent was distilled off under reduced pressure to obtain a crude product 2 (4.5 g). Sodium periodate (3.6 g, 0.017 mol) was added, while cooled with ice, to a tetrahydrofuran-water (60 ml, 1:1) solution of the crude product 2 (4.5 g, 0.013 mol), and the mixture was stirred for 45 minutes. Ethylene glycol (1.0 ml) was added, followed by stirring for 15 minutes, and then the mixture was extracted with ethyl acetate. The organic layer was washed with water, and a saturated aqueous solution of sodium chloride, and then dried over anhydrous sodium sulfate. The solvent was distilled off under reduced pressure to obtain a crude product 3 (4.0 g). A 37% aqueous formaldehyde solution (4.7 ml, 0.063 mol) and a 1 N aqueous sodium hydroxide solution (25 ml, 0.025 mol) were added to a tetrahydrofuran-water solution (60 ml, 1:1) of the resulting crude product 3, and the mixture was stirred for 18 hours at room temperature. After neutralization with dilute hydrochloric acid, the mixture was extracted with ethyl acetate. The organic layer was washed with water, and a saturated aqueous solution of sodium chloride, and then dried over anhydrous sodium sulfate. The solvent was distilled off under reduced pressure to obtain a crude product, which was purified by flash silica gel column chromatography (n-hexane:ethyl acetate = 1:1) to obtain Compound 4 (2.9 g, 3 steps 65%) as a colorless oily substance.
[α]_{D}²⁶-6.4 (c 0.82, CHCl₃).
IR λₘₐₓ (KBr): 3415, 2942, 1637, 1381, 1211, 1019 cm⁻¹.
¹H-NMR (CDCl₃) δ: 1.33 (3H, s), 1.65 (3H, s), 2.45 (1H, d, J = 7 Hz), 2.50 (1H, d, J = 7 Hz), 2.65 (1H, ddt, J = 6, 15, 2 Hz), 2.76 (1H, dd, J = 8, 15 Hz), 3.86, 3.90 (2H, ABX, J = 12, 6 Hz), 4.12, 4.17 (2H, ABX, J = 12, 7Hz), 4.54 (1H, d, J = 4 Hz), 4.65, 4.72 (2H, ABq, J = 10 Hz), 5.20-5.30 (2H, m), 5.72 (1H, d, J = 4 Hz), 5.88-6.04 (1H, m), 7.28-7.39 (5H, m).
¹³C-NMR (CDCl₃) δ: 25.8, 26.3, 37.0, 63.6, 64.0, 67.3, 83.1, 86.0, 87.4, 104.1, 112.6, 119.6, 127.4, 127.7, 128.3, 131.8, 137.9.
Mass (FAB) m/z: 373 (M+Na)⁺. High-resolution FAB-MS: Calcd. for C₁₉H₂₆O₆ (M+H)⁺ 373.3959; Found: 373.1613. Anal. Calcd. for C₁₈H₂₄O₆·1/10H₂O: C, 64.79; H, 7.50. Found: C, 64.52; H, 7.48%.

### (2) 3-C-Allyl-3,5-di-O-benzyl-4-C-hydroxymethyl-1,2-O-isopropylidene-α-D-ribofuranose (Compound 5)

In a stream of nitrogen, an anhydrous N,N-dimethylformamide solution (30 ml) of Compound 4 (2.5 g, 7.1 mmols) was added dropwise, while cooled with ice, to an anhydrous N,N-dimethylformamide suspension (30 ml) of 60% sodium hydride (400 mg, 9.9 mmols), followed by stirring the mixture for 1 hour. Benzyl bromide (4.0 ml, 7.4 mmols) was added, and the mixture was stirred for 1 hour at room temperature. After methanol was added, the solvent was distilled off under reduced pressure, and the residue was extracted with ether. The organic layer was washed with water, and a saturated aqueous solution of sodium chloride, and then dried over anhydrous magnesium sulfate. The solvent was distilled off under reduced pressure, and the resulting crude product was purified by flash silica gel column chromatography (chloroform:methanol = 30:1) to obtain Compound 5 (1.6 g, 52%) as a colorless oily substance.
[α]_{D}²⁵+14.2 (c 0.82, CHCl₃).
IR λₘₐₓ (KBr): 3564, 2936, 1637, 1380, 1106 cm⁻¹.
¹H-NMR (CDCl₃) δ: 1.31 (3H, s), 1.63 (3H, s), 2.24 (1H, dd, J = 5, 9 Hz), 2.49 (1H, dd, J = 6, 15 Hz), 2.79 (1H, dd, J = 7, 15 Hz), 3.57, 3.71 (2H, ABq, J = 10 Hz), 3.91 (1H, dd, J = 9, 12 Hz), 4.29 (1H, dd, J = 5, 12 Hz), 4.53 (1H, d, J = 4 Hz), 4.54, 4.60 (2H, ABq, J = 12 Hz), 4.66, 4.71 (2H, ABq, J = 11 Hz), 5.14-5.19 (2H, m), 5.71 (1H, d, J = 4 Hz), 5.84-5.99 (1H, m), 7.28-7.33 (10H, m).
¹³C-NMR (CDCl₃) δ: 25.9, 26.4, 36.4, 61.6, 67.8, 68.8, 73.7, 83.4, 85.5, 88.4, 103.7, 112.7, 119.2, 127.0, 127.2, 127.5, 127.6, 128.2, 132.2, 137.8, 138.3.
Mass (FAB) m/z: 447 (M+Li)⁺. High-resolution FAB-MS: Calcd. for C₂₆H₃₂O₆Na (M+Na)⁺ 463.5184; Found: 463.2093. Anal. Calcd. for C₂₆H₃₂O₆·1/2H₂O: C, 69.47; H, 7.40. Found: C, 69.06; H, 7.13%.

### (3) 3-C-Allyl-3,5-di-O-benzyl-1,2-O-isopropylidene-4-C-(p-toluenesulfonyloxymethyl)-α-D-ribofuranose (Compound 6)

In a stream of nitrogen, p-toluenesulfonyl chloride (1.0 g, 5.2 mmols), triethylamine (2 ml, 14 mmols), and N,N-dimethylaminopyridine (210 mg, 1.7 mmols) were added to an anhydrous methylene chloride solution (30 ml) of Compound 5 (1.5 g, 3.5 mmols), followed by stirring the mixture for 20 hours at room temperature. A saturated aqueous solution of sodium bicarbonate was added, the mixture was stirred for 30 minutes, and the system was extracted with ethyl acetate. The organic layer was washed with water, and a saturated aqueous solution of sodium chloride, and then dried over anhydrous sodium sulfate. The solvent was distilled off under reduced pressure, and the resulting crude product was purified by flash silica gel column chromatography (n-hexane:ethyl acetate = 4:1) to obtain Compound 6 (1.7 g, 80%) as a white powder. mp. 115-116°C.
[α]_{D}²⁵+17.2 (c 0.89, CHCl₃).
IR λₘₐₓ (KBr): 2936, 1361, 1177 cm⁻¹.
¹H-NMR (CDCl₃) δ: 1.27 (3H, s), 1.40 (3H, s), 2.31 (3H, s), 2.44 (1H, dd, J = 7, 15 Hz), 2.76 (1H, dd, J = 7, 15 Hz), 3.42, 3.65 (2H, ABq, J = 10 Hz), 4.37, 4.76 (2H, ABq, J = 10 Hz), 4.38, 4.41 (2H, ABq, J = 12 Hz), 4.50 (1H, d, J = 4 Hz), 4.59, 4.70 (2H, ABq, J = 12 Hz), 5.10-5.16 (2H, m), 5.65 (1H, d, J = 4 Hz), 5.76-5.92 (1H, m), 7.12-7.31 (12H, m), 7.69 (2H, d, J = 8 Hz).
¹³C-NMR (CDCl₃) δ: 21.6, 25.9, 26.0, 35.8, 66.3, 67.2, 69.2, 73.4, 83.5, 85.5, 86.7, 103.5, 113.0, 119.4, 126.6, 127.0, 127.4, 127.5, 128.0, 128.1, 128.2, 129.5, 131.7, 132.9, 137.5, 138.3, 144.2.
Mass (FAB) m/z: 617 (M+Na)⁺. Anal. Calcd. for C₃₃H₃₈O₈S: C, 66.65; H, 6.44; S, 5.39. Found: C, 66.49; H. 6.43; S. 5.31%.

### (4) 3,5-Di-O-benzyl-3-C-hydroxyethyl-1,2-O-isopropylidene-4-C-(p-toluenesulfonyloxymethyl)-α-D-ribofuranose (Compound 7)

Osmium tetroxide (0.07 M in t-butanol, 0.28 ml, 0.02 mmol) and sodium periodate (1.3 g, 6.0 mmols) were added, while cooled with ice, to a tetrahydrofuran-water solution (12 ml, 1:1) of Compound 6 (1.2 g, 2.0 mmols), whereafter the mixture was stirred for 2 hours at room temperature. After filtration, the system was extracted with ethyl acetate. The organic layer was washed with water, and a saturated aqueous solution of sodium chloride, and then dried over anhydrous sodium sulfate. The solvent was distilled off under reduced pressure, and sodium borohydride (75 mg, 2.0 mmols) was added, while cooled with ice, to a tetrahydrofuran-water solution (12 ml, 1:1) of the resulting crude product, followed by stirring the mixture for 1 hour at room temperature. After acetone was added, the mixture was extracted with ethyl acetate. The organic layer was washed with water, and a saturated aqueous solution of sodium chloride, and then dried over anhydrous sodium sulfate. The solvent was distilled off under reduced pressure, and the resulting crude product was purified by flash silica gel column chromatography (n-hexane:ethyl acetate = 1:1) to obtain Compound 7 (754 mg, 63%) as a colorless oily substance.
[α]_{D}²²+15.4 (c 0.83, CHCl₃).
IR λₘₐₓ (KBr): 3490, 2967, 1599, 1354 cm⁻¹.
¹H-NMR (CDCl₃) δ: 1.28 (3H, s), 1.41 (3H, s), 1.91 (1H, dt, J = 14, 7 Hz), 2.22 (1H, dt, J = 15, 6 Hz), 2.31 (3H, s), 3.38, 3.66 (2H, ABq, J = 10 Hz), 3.75 (1H, dd, J = 7, 11 Hz), 3.79 (1H, dd, J = 6, 11 Hz), 4.35, 4.79 (2H, ABq, J = 10 Hz), 4.39, 4.42 (2H, ABq, J = 11 Hz), 4.58, 4.69 (2H, ABq, J = 12 Hz), 4.66 (1H, d, J = 4 Hz), 5.71 (1H, d, J = 4 Hz), 7.14-7.35 (12H, m), 7.69 (2H, d, J = 8 Hz).
¹³C-NMR (CDCl₃) δ: 21.6, 25.9, 25.9, 33.8, 57.8, 66.4, 67.3, 69.1, 73.6, 83.8, 85.9, 86.7, 103.3, 113.2, 126.5, 127.1, 127.5, 127.6, 127.9, 128.1, 128.2, 129.5, 132.7, 137.3, 138.1, 144.3.
Mass (FAB) m/z: 621 (M+Na)⁺. Anal. Calcd. for C₃₂H₃₈O₉S·1/4H₂O: C, 63.72; H, 6.43; S, 5.36. Found: C, 63.79; H, 6.43: S, 5.13%.

### (5) 1,2-Di-O-acetyl-3-C-acetoxyethyl-3,5-di-O-benzyl-4-C-(p-toluenesulfonyloxymethyl)-D-ribofuranose (Compound 8)

Acetic anhydride (0.14 ml, 1.50 mmols) and concentrated sulfuric acid (1 drop) were added to an acetic acid solution (1.0 ml) of Compound 7 (140 mg, 0.23 mmol), whereafter the mixture was stirred for 6 hours at room temperature. After neutralization with sodium hydrogen carbonate, the system was extracted with ethyl acetate. The organic layer was washed with a saturated aqueous solution of sodium bicarbonate, water, and a saturated aqueous solution of sodium chloride, and then dried over anhydrous sodium sulfate. The solvent was distilled off under reduced pressure, and the resulting crude product was purified by flash silica gel column chromatography (n-hexane:ethyl acetate = 2:1) to obtain Compound 8 (154 mg, 98%) as a colorless oily substance.
Mass (FAB) m/z: 707 (M+Na)⁺. Anal. Calcd. for C₃₅H₄₀O₁₂S·1/5H₂O: C, 61.07; H, 5.92; S, 4.68. Found: C, 61.07; H, 5.94; S, 4.56%.

### (6) 2'-O-Acetyl-3'-C-acetyloxyethyl-3',5'-di-O-benzyl-5-methyl-4'-C-(p-toluenesulfonyloxymethyl)uridine (Compound 9)

In a stream of nitrogen, thymine (346 mg, 2.75 mmols) and N,O-bis(trimethylsilyl)acetamide (1.8 ml, 7.3 mmols) were added to an anhydrous 1,2-dichloroethane solution (25 ml) of Compound 8 (1.25 g, 1.83 mmols), whereafter the mixture was refluxed for 2 hours. The system was returned to room temperature, trimethylsilyltrifluoromethane sulfonate (0.56 ml, 3.1 mmols) was added, and the mixture was further refluxed for 20 hours. After a saturated aqueous solution of sodium bicarbonate was added, the mixture was extracted with ethyl acetate. The organic layer was washed with water and a saturated aqueous solution of sodium chloride, and then dried over anhydrous sodium sulfate. The solvent was distilled off under reduced pressure, and the resulting crude product was purified by flash silica gel column chromatography (n-hexane:ethyl acetate = 1:1) to obtain Compound 9 (1.20 g, 87%) as a white foamy substance.
mp. 68-72°C
[α]_{D}²¹-41.7 (c 0.79, CHCl₃).
IR λₘₐₓ (KBr): 3033, 1746, 1693, 1367, 1230 cm⁻¹.
¹H-NMR (CDCl₃) δ: 1.42 (3H, d, J = 1 Hz), 2.03 (3H, s), 2.09 (3H, s), 2.42 (3H, s), 2.34-2.42 (2H, m), 3.85, 3.92 (2H, ABq, J = 11 Hz), 4.09, 4.23 (2H, ABq, J = 11 Hz), 4.11-4.22 (2H, m), 4.63, 4.73 (2H, ABq, J = 12 Hz), 4.64, 5.00 (2H, ABq, J = 12 Hz), 5.79 (1H, d, J = 9 Hz), 6.21 (1H, d, J = 9 Hz), 7.20-7.56 (12H, m), 7.67 (1H, s), 7.68 (2H, d, J = 6Hz), 7.95 (1H, brs).
¹³C-NMR (CDCl₃) δ: 11.9, 20.8, 21.0, 21.8, 28.1, 59.4, 67.0, 70.0, 71.2, 73.9, 77.6, 83.1, 83.1, 87.3, 111.4, 126.8, 127.3, 127.6, 127.9, 128.2, 128.4, 128.7, 129.8, 131.6, 135.5, 136.2, 137.4, 145.1, 150.6, 163.1, 169.5, 170.6. Mass (FAB) m/z: 751 (M+H)⁺. Anal. Calcd. for C₃₈H₄₂O₁₂N₂S: C, 60.43; H, 5.67; N, 3.71; S, 4.27. Found: C, 60.43; H, 5.67; N, 3.77; S, 4.03%.

### (7) 3'-C-Acetyloxyethyl-3',5'-di-O-benzyl-5-methyl-4'-C-(p-toluenesulfonyloxymethyl)uridine (Compound 10)

A 40% aqueous solution (1.5 ml, 18 mmols) of methylamine was added, while cooled with ice, to a tetrahydrofuran solution (25 ml) of Compound 9 (1.32 g, 1.76 mmols), and the mixture was stirred for 1 hour. A 40% aqueous methylamine solution (1.5 ml, 18 mmols) was added, and stirred for a further 1 hour. The solvent was distilled off under reduced pressure, and then the residue was extracted with ethyl acetate. The organic layer was washed with water and a saturated aqueous solution of sodium chloride, and then dried over anhydrous sodium sulfate. The solvent was distilled off under reduced pressure, and the resulting crude product was purified by flash silica gel column chromatography (chloroform:methanol = 40:1) to obtain Compound 10 (1.08 g, 87%) as a white foamy substance.
mp. 69-71°C
[α]_{D}²¹-10.6 (c 0.73, CHCl₃).
IR λₘₐₓ (KBr): 3381, 3064, 1741, 1704, 1366, 1234 cm⁻¹.
¹H-NMR (CDCl₃) δ: 1.49 (3H, s), 2.08 (3H, s), 2.40 (3H, s), 2.40-2.47 (2H, m), 3.72, 3.81 (2H, ABq, J = 11 Hz), 4.06, 4.20 (2H, ABq, J = 10 Hz), 4.38 (1H, d, J = 8 Hz), 4.29-4.40 (2H, m), 4.54 (1H, d, J = 11 Hz), 4.61-4.65 (2H, m), 4.88 (1H, d, J = 11 Hz), 5.89 (1H, d, J = 8 Hz), 7.16-7.36 (12H, m), 7.49 (1H, s), 7.69 (2H, d, J = 8 Hz), 8.20 (1H, brs).
¹³C-NMR (CDCl₃) δ: 12.0, 21.1, 21.8, 28.9, 60.0, 69.5, 71.7, 74.0, 79.1, 83.6, 86.7, 87.6, 111.2, 127.0, 127.6, 127.7, 127.9, 128.3, 128.4, 128.7, 129.9, 131.5, 135.6, 136.3, 137.1, 145.3, 155.0, 163.2, 170.7.
Mass (FAB) m/z: 709 (M+H)⁺. High-resolution FAB-MS: Calcd. for C₃₆H₄₁N₂O₁₁S (M+H)⁺ 709.7836; Found: 709.2424.

### (8) 3'-C-Acetyloxyethyl-3',5'-di-O-benzyl-2'-O-(2-methoxypropyl)-5-methyl-4'-C-(p-toluenesulfonyloxymethyl)uridine (Compound 11)

2-Methoxy-2-propene (0.067 ml, 0.7 mmol) and p-toluenesulfonic acid monohydrate (1.0 mg, 0.06 mmol) were added, while cooled with ice, to an anhydrous dichloromethane solution (3.0 ml) of Compound 10 (197 mg, 0.28 mmol), and the mixture was stirred for 1.5 hours. After a saturated aqueous solution of sodium bicarbonate was added, the mixture was extracted with ethyl acetate. The organic layer was washed with water and a saturated aqueous solution of sodium chloride, and then dried over anhydrous sodium sulfate. The solvent was distilled off under reduced pressure, and the resulting crude product was purified by flash silica gel column chromatography (n-hexane:ethyl acetate = 1:1) to obtain Compound 11 (186 mg, 85%) as a white foamy substance.
mp. 72-74°C.
[α]_{D}²⁵-37.6 (c 0.71, CHCl₃).
IR λₘₐₓ (KBr): 2989, 1692, 1367, 1235, 1178 cm⁻¹.
¹H-NMR (CDCl₃) δ: 1.24 (6H, s), 1.49 (3H, s), 2.07 (3H, s), 2.20-2.28 (1H, m), 2.42 (3H, s), 2.53-2.61 (1H, m), 3.00 (3H, s), 3.88, 3.94 (2H, ABq, J = 11 Hz), 4.10 (1H, d, J = 11 Hz), 4.19-4.26 (3H, m), 4.55-4.62 (3H, m), 4.68 (1H, d, 11 Hz), 5.18 (1H, d, J = 11 Hz), 6.14 (1H, d, J = 8 Hz), 7.21-7.36 (12H, m), 7.49 (1H, s), 7.69 (2H, d, J = 8 Hz), 8.06 (1H, s).
¹³C-NMR (CDCl₃) δ: 11.9, 21.1, 21.8, 24.7, 25.3, 27.9, 50.1, 59.8, 67.0, 70.7, 71.9, 74.0, 78.5, 83.4, 84.0, 86.0, 101.9, 110.8, 126.8, 127.3, 127.7, 127.9, 128.3, 128.3, 128.7, 129.7, 131.9, 136.5, 136.6, 138.3, 145.0, 150.7, 163.2, 170.7.
Mass (FAB) m/z: 781 (M+H)⁺. Anal. Calcd. for C₄₀H₄₈O₁₂N₂S·1/5H₂O: C, 61.24; H, 6.22; N, 3.57; S, 3.93.
Found: C, 61.20; H, 6.15; N, 3.49; S, 4.11%.

### (9) 3',5'-Di-O-benzyl-3'-C-hydroxyethyl-2'-O-(2-methoxypropyl)-5-methyl-4'-C-(p-toluenesulfonyloxymethyl)uridine (Compound 12)

A 2N aqueous solution (1.3 ml, 2.6 mmols) of sodium hydroxide was added to a tetrahydrofuran-methanol solution (18 ml, 3:1) of Compound 11 (974 mg, 1.25 mmols), and the mixture was stirred for 1.5 hours at room temperature. After water was added to dilute the mixture, the system was extracted with dichloromethane. The organic layer was washed with water and a saturated aqueous solution of sodium chloride, and then dried over anhydrous sodium sulfate. The solvent was distilled off under reduced pressure, and the resulting crude product was purified by flash silica gel column chromatography (n-hexane:ethyl acetate = 1:1) to obtain Compound 12 (806 mg, 87%) as a white foamy substance.
mp. 90-92°C.
[**α**]_{D}²⁵-52.3 (c 0.90, CHCl₃).
IR **λ**ₘₐₓ (KBr): 3417, 3190, 2990, 1692, 1364, 1176 cm⁻¹.
¹H-NMR (CDCl₃) δ: 1.24 (3H, s), 1.27 (3H, s), 1.48 (3H, d, J = 1 Hz), 1.68 (1H, brs), 2.04-2.18 (1H, m), 2.43 (3H, s), 2.51-2.57 (1H, m), 3.00 (3H, s), 3.77-3.81 (2H, m), 3.84, 4.01 (2H, ABq, J = 11 Hz), 4.14, 4.37 (2H, ABq, J = 11 Hz), 4.54, 5.15 (2H, ABq, J = 12 Hz), 4.58, 4.65 (2H, ABq, J = 11 Hz), 4.64 (1H, d, J = 8 Hz), 6.12 (1H, d, J = 8 Hz), 7.17-7.39 (12H, m), 7.50 (1H, d, J = 1 Hz), 7.69 (2H, d, J = 8 Hz), 8.06 (1H, brs).
¹³C-NMR (CDCl₃) δ: 11.9, 21.8, 24.7, 25.3, 31.2, 50.0, 57.7, 66.8, 71.2, 71.8, 74.0, 78.4, 84.0, 84.2, 86.1, 101.8, 110.8, 126.7, 127.2, 127.6, 127.9, 128.2, 128.6, 129.7, 132.0, 136.69, 136.72, 138.6, 144.9, 150.7, 163.3.
Mass (FAB) m/z: 739 (M+H)⁺. Anal. Calcd. for C₃₈H₄₆O₁₁N₂S·1/2H₂O: C, 61.03; H, 6.33; N, 3.75: S, 4.34.
Found: C, 61.06; H, 6.25; N, 3.62; S, 4.24%.

### (10) 3',5'-Di-O-benzyl-2'-O-(2-methoxypropyl)-5-methyl-3'-C,4'-C-ethoxymethyleneuridine (Compound 13)

In a stream of nitrogen, sodium bistrimethylsilylamide (1.0 M in THF, 3.0 ml, 3.0 mmols) was added to an anhydrous tetrahydrofuran solution (16 ml) of Compound 12 (728 mg, 0.99 mmol), and the mixture was refluxed for 6 hours. A saturated aqueous solution of sodium bicarbonate was added, and the mixture was extracted with ethyl acetate. The organic layer was washed with water and a saturated aqueous solution of sodium chloride, and then dried over anhydrous sodium sulfate. The solvent was distilled off under reduced pressure, and the resulting crude product was recrystallized from ethyl acetate-n-hexane to obtain Compound 13 (400 mg, 73%) as a white powder.
mp. 172-175°C.
[α]_{D}²⁶-129.8 (c 0.81, CHCl₃).
IR λₘₐₓ (KBr): 3178, 2955, 1695, 1460, 1088 cm⁻¹.
¹H-NMR (CDCl₃) δ: 1.29 (3H, s), 1.38 (3H, s), 1.56 (3H, s), 1.93-2.05 (1H, m), 2.30-2.35 (1H, m), 3.01 (3H, s), 3.25 (1H, d, J = 10 Hz), 3.77-3.85 (3H, m), 4.29-4.36 (2H, m), 4.58, 5.33 (2H, ABq, J = 11 Hz), 4.67 (2H, s), 5.42 (1H, d, J = 8 Hz). 6.56 (1H, d, 8 Hz), 7.26-7.47 (10H, m), 7.96 (1H, s).
¹³C-NMR (CDCl₃) δ: 12.1, 24.4, 26.4, 27.8, 49.0, 62.1, 66.1, 66.4, 73.9, 75.5, 76.5, 79.1, 83.6, 86.6, 101.0, 110.6, 127.3, 127.5, 127.7, 128.1, 128.3, 128.6, 136.6, 137.5, 138.4, 151.1, 163.4.
Mass (FAB) m/z: 567 (M+H)⁺. Anal. Calcd. for C₃₁H₃₈O₈N₂·1/3H₂O: C, 65.02; H, 6.81; N, 4.89. Found: C, 64.98; H, 6.81; N, 4.83%.

### (11) 3',5'-Di-O-benzyl-5-methyl-3'-C,4'-C-ethoxymethyleneuridine (Compound 14)

p-Toluenesulfonic acid monohydrate (64 mg, 0.34 mmol) was added, while cooled with ice, to a tetrahydrofuran-methanol solution (9.0 ml, 2:1) of Compound 13 (466 mg, 0.84 mmol), and the mixture was stirred for 1 hour. After a saturated aqueous solution of sodium bicarbonate was added, the mixture was extracted with ethyl acetate. The organic layer was washed with water and a saturated aqueous solution of sodium chloride, and then dried over anhydrous sodium sulfate. The solvent was distilled off under reduced pressure, and the resulting crude product was purified by flash silica gel column chromatography (n-hexane:ethyl acetate = 1:1) to obtain Compound 14 (355 mg, 85%) as a white powder.
mp. 79-82°C.
[α]_{D}²⁷-45.5 (c 0.66, CHCl₃).
IR λₘₐₓ (KBr): 3429, 1687, 1489, 1271, 1071 cm⁻¹.
¹H-NMR (CDCl₃) δ: 1.77 (3H, s), 1.80-1.87 (1H, m), 2.44-2.50 (1H, brd, J = 15 Hz), 3.53, 3.97 (2H, ABq, J = 10 Hz), 3.69 (1H, dt, J = 2, 12 Hz), 3.89, 4.33 (2H, ABq, J = 10 Hz), 3.86 (1H, m), 4.48 (2H, s), 4.63, 5.38 (2H, ABq, J = 11 Hz), 4.66 (1H, d, J = 5 Hz), 5.03 (1H, s), 5.72 (1H, d, J = 5 Hz), 7.24-7.43 (10H, m), 7.86 (1H, s), 9.06 (1H, brs).
¹³C-NMR (CDCl₃) δ: 12.5, 27.7, 62.4, 65.1, 66.7, 71.0, 73.7, 80.0, 83.0, 86.0, 94.5, 109.9, 127.1, 127.2, 127.9, 128.2, 128.4, 136.4, 137.0, 138.9, 152.1, 163.7.
Mass (FAB) m/z: 495 (M+H)⁺. High-resolution FAB-MS: Calcd. for C₂₇H₃₀O₇N₂ (M+H)⁺ 495.5443; Found: 495.2137.

### (12) 5-Methyl-3'-C,4'-C-ethoxymethyleneuridine (Compound 15)

20% Palladium hydroxide/carbon powder (17 mg) and cyclohexene (0.35 ml, 3.5 mmols) were added to an ethanol solution (1.5 ml) of Compound 14 (34 mg, 0.07 mmol), and the mixture was refluxed for 6 hours. Cyclohexene was further added, and the mixture was further refluxed for 3 hours. After filtration, the solvent was distilled off under reduced pressure. The resulting crude product was purified by flash silica gel column chromatography (chloroform:methanol = 20:1) to obtain Compound 15 (15 mg, 69%) as a white powder.
mp. 244-246°C.
[α]_{D}²⁷-42.5 (c 0.69, CH₃OH).
IR λₘₐₓ (KBr): 3449, 3251, 2972, 2885, 1700, 1468, 1007 cm⁻¹. ¹H-NMR (CDCl₃) δ: 1.76 (1H, dd, J = 3, 13 Hz), 1.85-1.96 (1H, m), 1.88 (3H, J = 1 Hz), 3.58 (1H, d, J = 9 Hz), 3.77 (1H, dd, J = 5, 11 Hz), 3.86 (1H, d, J = 12 Hz), 3.96-4.08 (3H, m), 4.73 (1H, d, J = 7 Hz), 5.98 (1H, d, J = 7 Hz), 8.06 (1H, d, J = 1 Hz).
¹³C-NMR (CDCl₃) δ: 12.6, 32.3, 63.2, 64.2, 65.9, 76.9, 77.5, 85.3, 92.2, 111.2, 139.4, 153.4, 166.3.
Mass (EI) m/z: 314 (M⁺, 16.5).

### [Example 2] Synthesis of nucleoside analogue: Synthesis of 3'-O-[2-cyanoethoxy(diisopropylamino)phosphino]-5'-0-(4,4'-dimethoxytrityl)-5-methyl-2'-O-methyl-3'-C,4'-C-ethoxymethyleneuridine (Compound 20)

### (1) 5-Methyl-3-N-benzyloxymethyl-3',5'-di-O-benzyl-3'-C,4'-C-ethoxymethyleneuridine (Compound 16)

In a stream of nitrogen, 1,8-diazabicyclo[4.3.0]non-7-ene (63 µl, 0.42 mmol) and benzylchloromethyl ether (44 µl, 0.32 mmol) were added, while cooled with ice, to an anhydrous N,N-dimethylformamide solution (2 ml) of Compound 14 (106 mg, 0.21 mmol), followed by stirring the mixture for 45 minutes. Methanol was added, and the mixture was extracted with ethyl acetate. The organic layer was washed with water and a saturated aqueous solution of sodium chloride, and then dried over anhydrous sodium sulfate. The solvent was distilled off under reduced pressure, and the resulting crude product was purified by flash silica gel column chromatography (n-hexane:ethyl acetate = 2:1) to obtain Compound 16 (116 mg, 90%) as a white foamy substance. mp. 48-49°C.
[α]_{D}²⁶-32.9 (c 0.77, CHCl₃).
IR λₘₐₓ (KBr): 3430, 2954, 2874, 1698, 1657, 1458, 1071 cm⁻¹. ¹H-NMR (CDCl₃) δ: 1.77 (3H, s), 1.77-1.85 (1H, m), 2.47 (1H, d, J = 14 Hz), 3.41, 3.97 (2H, ABq, J = 10 Hz), 3.65-3.73 (1H, m), 3.84-3.88 (1H, m), 3.92, 4.31 (2H, ABq, J = 10 Hz), 4.47 (2H, s), 4.54 (1H, d, J = 5 Hz), 4.62, 5.39 (2H, ABq, J = 11 Hz), 4.70 (2H, s), 5.11 (1H, s), 5.45, 5.49 (2H, ABq, J = 9 Hz), 5.69 (1H, d, J = 5 Hz), 7.25-7.44 (16H, m), 7.83 (1H, s).
¹³C-NMR (CDCl₃) δ: 13.3, 27.8, 62.4, 64.9, 66.8, 70.6, 70.7, 72.4, 73.6, 80.0, 83.2, 86.0, 94.8, 109.4, 127.1, 127.2, 127.3, 127.5, 127.6, 127.8, 128.2, 128.2, 128.4, 134.8, 137.1, 137.9, 138.9, 152.8, 163.0.
Mass (FAB) m/z: 615 (M+H)⁺. Anal. Calcd. for C₃₅H₃₈O₈N₂·1/10H₂O: C, 68.19; H, 6.23; N, 4.54. Found: C, 68.01; H, 6.25; N, 4.49%.

### (2) 3-N-Benzyloxymethyl-3',5'-di-O-benzyl-2'-O-methyl-5-methyl-3'-C,4'-C-ethoxymethyleneuridine (Compound 17)

In a stream of nitrogen, an anhydrous N,N-dimethylformamide solution (1.4 ml) of Compound 16 (109 mg, 0.18 mmol) was added dropwise, while cooled with ice, to an anhydrous N,N-dimethylformamide suspension (0.6 ml) of 60% sodium hydride (9 mg, 0.23 mmol), followed by stirring the mixture for 30 minutes. Iodomethane (60 µl, 0.9 mmol) was added, and the mixture was stirred for 1 hour at room temperature. After methanol was added, the solvent was distilled off under reduced pressure, and the residue was extracted with ethyl acetate. The organic layer was washed with water and a saturated aqueous solution of sodium chloride, and then dried over anhydrous magnesium sulfate. The solvent was distilled off under reduced pressure, and the resulting crude product was purified by flash silica gel column chromatography (n-hexane:ethyl acetate = 2:1) to obtain Compound 17 (97 mg, 86%) as a white foamy substance. mp. 44-45°C.
[α]_{D}²⁴-89.1 (c 0.80, CHCl₃).
IR λₘₐₓ (KBr): 2953, 2885, 1712, 1669, 1456, 1090 cm⁻¹.
¹H-NMR (CDCl₃) δ: 1.65 (3H, s), 2.02-2.14 (1H, m), 2.45 (1H, brd, J = 14 Hz), 3.31, 4.33 (2H, ABq, J = 10 Hz), 3.44 (3H, s), 3.78 (2H, d, J = 8 Hz), 3.86, 4.28 (2H, ABq, J = 11 Hz), 4.65-4.67 (5H, m), 4.87 (1H, d, J = 8 Hz), 5.29 (1H, d, J = 11 Hz), 5.47, 5.50 (2H, ABq, J = 10 Hz), 6.55 (1H, d, J = 8 Hz), 7.23-7.45 (15H, m), 7.80 (1H, s).
¹³C-NMR (CDCl₃) δ: 13.1, 27.6, 59.4, 61.8, 66.1, 66.2, 70.7, 72.1, 73.9, 74.9, 79.1, 83.5, 86.6, 87.7, 110.3, 127.3, 127.3, 127.4, 127.6, 128.1, 128.2, 128.3, 128.6, 135.5, 136.8, 137.9, 138.4, 151.8, 163.3.
Mass (FAB) m/z: 629 (M+H)⁺. Anal. Calcd. for C₃₆H₄₀O₈N₂·1/5H₂O: C, 68.38; H, 6.44; N, 4.43. Found: C, 68.31; H, 6.43; N, 4.45%.

### (3) 5-Methyl-2'-O-methyl-3'-C,4'-C-ethoxymethyleneuridine (Compound 18)

20% Palladium hydroxide/carbon powder (50 mg) and cyclohexene (0.7 ml, 7 mmols) were added to an ethanol solution (2 ml) of Compound 17 (91 mg, 0.14 mmol), and the mixture was refluxed for 7 hours. After filtration through a fluted filter, the solvent was distilled off under reduced pressure. The resulting crude product was purified by flash silica gel column chromatography (chloroform:methanol = 20:1) to obtain Compound 18 (32 mg, 70%) as a white powder.

X-ray structural analysis of Compound 8 showed that this compound had a stereostructure very similar to that of a DNA molecule.
mp. 256-259°C.
[α]_{D}²⁷-49.7 (c 0.53, CH₃OH).
IR λₘₐₓ (KBr): 3238, 2966, 1705, 1469 cm⁻¹.
¹H-NMR (CD₃OD) δ: 1.84 (1H, dd, J = 3, 13 Hz), 1.90 (3H, d, J = 1 Hz), 2.07 (1H, dt, J = 13, 6 Hz), 3.42 (3H, s), 3.47 (1H, d, J = 9 Hz), 3.75 (1H, dd, J = 5, 11 Hz), 3.95-4.08 (4H, m), 4.63 (1H, d, J = 7 Hz), 6.17 (1H , d, J = 7 Hz), 8.10 (1H, d, J = 1 Hz).
¹³C-NMR (CDCl₃) δ: 12.6, 33.5, 59.2, 63.0, 64.7, 65.9, 84.7, 85.7, 89.7, 111.8, 139.5, 153.0, 166.1.
Mass (FAB) m/z: 329 (M+H)⁺. Anal. Calcd. for C₁₄H₂₀O₇N₂: C, 51.22; H, 6.14; N, 8.53. Found: C, 51.25; H, 6.12; N, 8.31%.

### (4) 5'-O-(4,4'-Dimethoxytrityl)-5-methyl-2'-O-methyl-3'-C,4'-C-ethoxymethyleneuridine (Compound 19)

In a stream of nitrogen, 4,4'-dimethoxytrityl chloride (70 mg, 0.21 mmol) was added to an anhydrous pyridine solution (1.0 ml) of Compound 18 (34 mg, 0.10 mmol), and the mixture was stirred for 3.5 hours at 60°C. A saturated aqueous solution of sodium bicarbonate was added, and the mixture was extracted with ethyl acetate. The organic layer was washed with water and a saturated aqueous solution of sodium chloride, and then dried over anhydrous magnesium sulfate. The solvent was distilled off under reduced pressure, and the resulting crude product was purified by flash silica gel column chromatography (n-hexane:ethyl acetate = 1:4) to obtain Compound 19 (51 mg, 81%) as a white foamy substance.
mp. 127-129°C.
[α]_{D}²⁴-49.4 (c 0.91, CHCl₃).
IR λₘₐₓ (KBr): 3458, 3195, 3007, 2953, 2837, 1691, 1508, 1464, 1254 cm⁻¹.
¹H-NMR (CDCl₃) δ: 1.28 (3H, s), 1.45-1.53 (2H, m), 2.94 (2H, s), 3.40-3.48 (2H, m), 3.51 (2H, s), 3.80 (6H, s), 3.91 (1H, dt, J = 4, 10 Hz), 4.09 (1H, d, J = 9 Hz), 4.74 (1H, d, J = 6 Hz), 6.10 (1H, d, J = 6 Hz), 6.84 (4H, dd, J = 2, 8 Hz), 7.24-7.39 (9H, m), 7.98 (1H, d, J = 1 Hz), 8.18 (1H, brs). ¹³C-NMR (CDCl₃) δ: 11.4, 32.4, 55.3, 59.2, 61.8, 65.8, 65.9, 75.8, 83.8, 84.3, 88.3, 88.5, 111.3, 113.1, 113.1, 127.5, 127.9, 128.8, 130.7, 130.7, 134.2, 134.4, 137.3, 142.2, 158.9.
Mass (FAB) m/z: 631 (M+H)⁺. Anal. Calcd. for C₃₅H₃₈O₉N₂·1/2H₂O: C, 65.72; H, 6.14; N, 4.38. Found: C, 65.72; H, 6.15; N, 4.31%.

### (5) 3'-O-[2-Cyanoethoxy(diisopropylamino)phosphino]-5'-O-(4,4'-dimethoxytrityl)-5-methyl-2'-O-methyl-3'-C,4'-C-ethoxymethyleneuridine (Compound 20)

4,5-Dicyanoimidazole (37 mg, 0.31 mmol) and 2-cyanoethyl N,N,N',N'-tetraisopropylphosphorodiamidite (112 µl, 0.36 mmol) were added to an anhydrous acetonitrile solution (1.0 ml) of Compound 19 (50 mg, 0.08 mmol). The mixture was stirred for 21 hours at room temperature, and then stirred for 3 hours at 50°C. A saturated aqueous solution of sodium bicarbonate was added, and the mixture was extracted with ethyl acetate. The organic layer was washed with a saturated aqueous solution of sodium bicarbonate and a saturated aqueous solution of sodium chloride, and then dried over anhydrous sodium sulfate. The solvent was distilled off under reduced pressure, and the residue was purified by flash silica gel column chromatography (n-hexane:ethyl acetate = 2:3). The resulting crude product was purified again by reprecipitation (n-hexane-ethyl acetate) to obtain Compound 20 (3 mg, 5%) as a white powder.
mp. 115-118°C.
³¹P-NMR (CDCl₃) δ: 143.4, 146.9.
Mass (FAB) m/z: 853 (M+Na)⁺. High-resolution FAB-MS: Calcd. for C₄₄H₅₆O₁₀N₄P (M+H)⁺ 831.9102; Found: 831.3730.

### [Example 3] Synthesis of nucleoside analogue:

The compounds of the present invention with a hydrogen atom at the 2'-position can be synthesized, for example, in accordance with the following synthesis scheme:

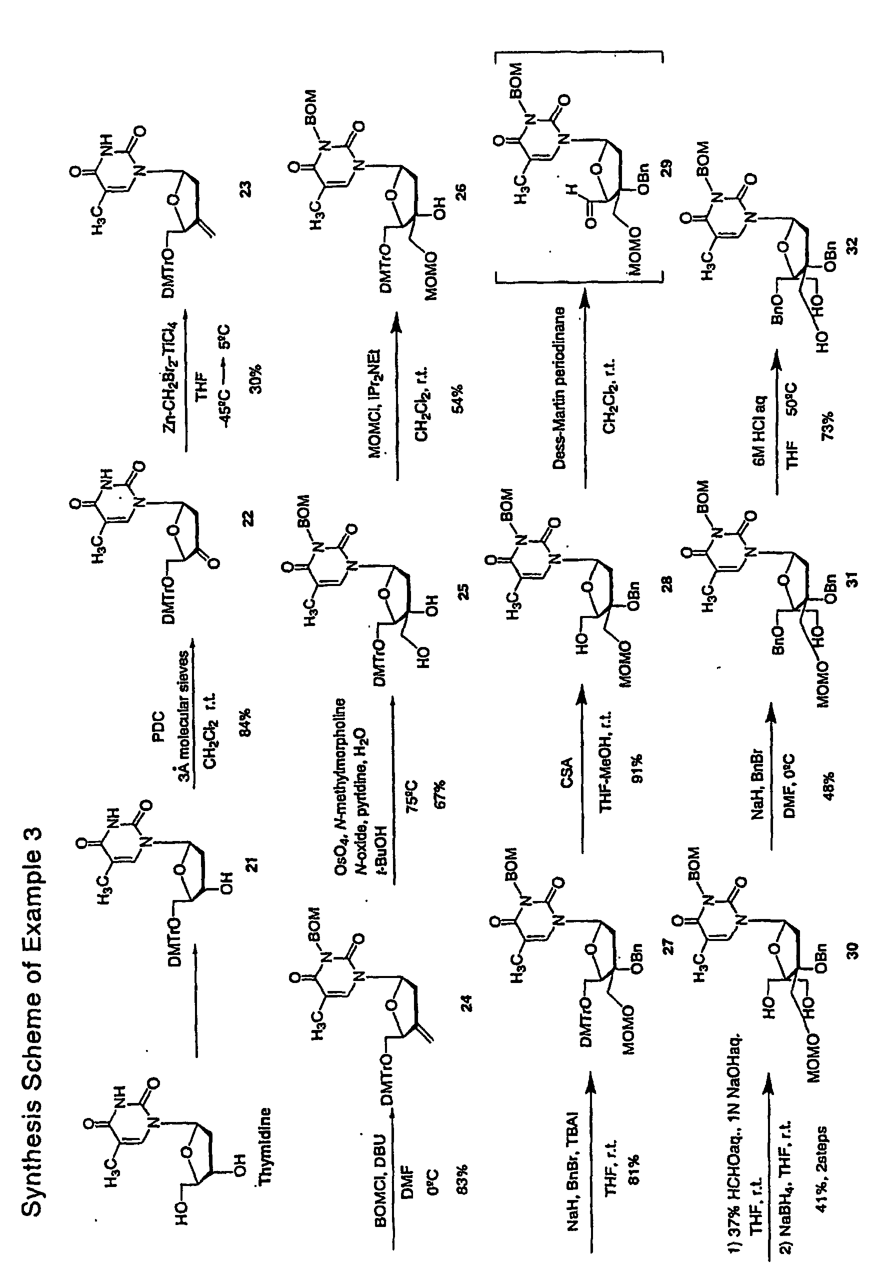

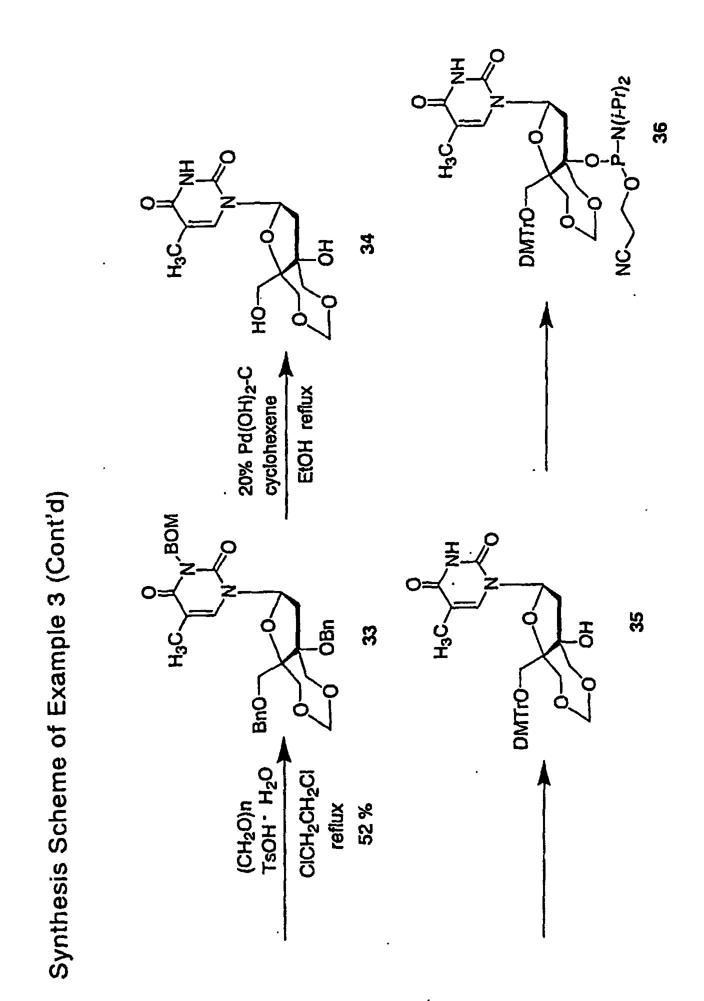

### Synthesis of 3'-C,4'-C-[methylenebis(oxymethyl)]thymidine (Compound 34)

### (1) 3'-Keto-5'-O-(4,4'-dimethoxytrityl)thymidine (Compound 22)

Synthesized in the following manner according to literature [Nucleosides & Nucleotides 14, 921-924 (1995)]: In a stream of nitrogen, Compound 21 (22 g, 40 mmols; Compound 21 can be obtained, with thymidine as the starting material, by the same method as in Example 4 to be described later) was added to an anhydrous dichloromethane suspension (250 ml) of pyridinium dichromate (PDC) (18 g, 49 mmols) and a 3Å molecular sieve powder (18 g, Sigma M1885), and the mixture was stirred for 3 hours at room temperature. After filtration using a glass filter thinly covered with a 3Å molecular sieve powder, the mother liquor was distilled off under reduced pressure. Ethyl acetate was added to the resulting crude product, and the mixture was dissolved upon exposure to ultrasonic waves for 5 minutes. Then, the solution was filtered with the use of a glass filter thinly covered with a 3Å molecular sieve powder. The motor liquor was distilled off under reduced pressure to obtain Compound 22 (18.3 g, 84%) as a brown foamy substance.
¹H-NMR (CDCl₃) δ: 1.34 (3H, s), 2.74 (1H, dd, J = 8, 18 Hz), 3.08 (1H, dd, J = 7, 18 Hz), 3.41-3.45 (1H, m), 3.63 (1H, dd, J = 2, 10 Hz), 3.78 (6H, s), 4.16 (1H, brs), 6.58 (1H, t, J = 7 Hz), 6.81-7.36 (13H, m), 7.65 (1H, s), 9.31 (1H, s).

### (2) 3'-Deoxy-5'-O-(4,4'-dimethoxytrityl)-3'-methylenethymidine (Compound 23)

Synthesized in the following manner according to literature [Nucleosides & Nucleotides 14, 921-924 (1995)]: In a stream of nitrogen, an anhydrous dichloromethane solution (220 ml) of Compound 22 (18.3 g, 34 mmols) was poured, at one stroke at 5°C, into a reagent, which had been prepared according to the literature from zinc (41 g, 0.63 mol), dibromomethane (14.3 ml, 0.13 mol), titanium tetrachloride (16.7 ml, 0.15 mol) and anhydrous tetrahydrofuran (353 ml), whereafter the mixture was stirred as such for 2 hours. The reaction mixture was added, little by little, into a saturated aqueous solution of sodium bicarbonate for neutralization, and then the mixture was filtered through a glass filter spread with silica gel. The mother liquor was distilled off under reduced pressure, and then the residue was extracted with chloroform. The extract was washed with water and a saturated aqueous solution of sodium chloride, and then dried over anhydrous sodium sulfate. The solvent was distilled off under reduced pressure, and the residue was purified by flash silica gel column chromatography (ethyl acetate:n-hexane = 1:1) to obtain Compound 23 (6.4 g, 30%) as a yellow foamy substance.
¹H-NMR (CDCl₃) δ: 1.46 (3H, s), 2.81 (1H, dd, J= 6, 15 Hz), 3.11 (1H, dd, J = 6, 16 Hz), 3.38-3.40 (2H, m), 3.78 (6H, s), 4.58 (1H, brs), 4.98 (1H, brs), 5.23 (1H, brs), 6.28 (1H, t, J = 7 Hz), 6.81-7.45 (13H, m), 7.62 (1H. s), 9.39 (1H, brs).

### (3) 3-N-Benzyloxymethyl-3'-deoxy-5'-O-(4,4'-dimethoxytrityl)-3'-methylenethymidine (Compound 24)

In a stream of nitrogen, 1,8-diazabicyclo[5.4.0]undeca-7-ene (DBU) (2.4 ml, 16 mmols) and benzylchloromethyl ether (1.3 ml, 9.6 mmols) were added, while cooled with ice, to an anhydrous N,N-dimethylformamide solution (60 ml) of Compound 23 (4.34 g, 8 mmols), followed by stirring the mixture for 2.5 hours at room temperature. Methanol was added, and the solvent was distilled off under reduced pressure. The residue was extracted with ethyl acetate, and then the extract was washed with water and a saturated aqueous solution of sodium chloride, and then dried over anhydrous sodium sulfate. The solvent was distilled off under reduced pressure, and the resulting crude product was purified by column chromatography (ethyl acetate:n-hexane = 1:2) to obtain Compound 24 (4.4 g, 83%) as a white foamy substance. ¹H-NMR (CDCl₃) δ: 1.44 (3H, s), 2.74-2.84 (1H,m), 3.07-3.16(1H, m), 3.38 (2H, brs), 3.79 (6H, s), 4.59 (1H, brs), 4.71 (2H, s), 4.99 (1H, brs), 5.25 (1H, brs), 5.49 (2H, s), 6.30 (1H, t, J = 7 Hz), 6.82 (4H, d, J = 9 Hz), 7.21-7.44 (14H, m), 7.62 (1H, s).

### (4) 3-N-Benzyloxymethyl-3'-C-hydroxymethyl-5'-O-(4,4'-dimethoxytrityl)thymidine (Compound 25)

In a stream of nitrogen, N-methylmorpholine N-oxide (NMO) (5.5 g, 47 mmols), pyridine (3.3 ml, 40 mmols), H₂O (4.3 ml) and osmium tetroxide (0.07 M tert-butanol solution, 0.47 ml, 33 mmols) were added to a tert-butanol solution (100 ml) of Compound 24 (4.4 g, 6.7 mmols), followed by stirring the mixture for 7 hours at 76°C. The reaction mixture was cooled to room temperature, and a 20% aqueous solution (15 ml) of sodium thiosulfate was added, followed by stirring the mixture for 15 minutes. The solvent was distilled off under reduced pressure. The residue was extracted with ethyl acetate, and the extract was washed with a 0.5 M aqueous solution of potassium bisulfate, water and a saturated aqueous solution of sodium chloride, and then dried over anhydrous sodium sulfate. The solvent was distilled off under reduced pressure, and the resulting crude product was purified by column chromatography (ethyl acetate:chloroform = 1:1) to obtain Compound 25 (3.13 g, 67%) as a white foamy substance.
¹H-NMR (CDCl₃) δ: 1.51 (3H, d, J = 1 Hz), 1.94 (1H, dd, J = 9, 13 Hz), 2.34 (1H, dd, J = 5, 13 Hz), 2.54 (1H, brt), 2.96 (1H, brd), 3.32 (1H, dd, J = 2, 11 Hz), 3.46 (1H, dd, J = 5, 11 Hz), 3.69 (2H, d, J = 6 Hz), 3.80 (6H, s), 4.10 (1H, m), 4.70 (2H, s), 5.48 (2H, s), 6.42 (1H, dd, J = 5, 9 Hz), 6.85 (4H, d, J = 9 Hz), 7.27-7.40 (14H, m), 7.48 (1H, s).

### (5) 3-N-Benzyloxymethyl-3'-C-methoxymethyloxymethyl-5'-O-(4,4'-dimethoxytrityl)thymidine (Compound 26)

In a stream of nitrogen, N-ethyldiisopropylamine (0.32 ml, 2.1 mmols) and chloromethyl methyl ether (0.14 ml, 1.04 mmols) were added, while cooled with ice, to an anhydrous dichloromethane solution (5 ml) of Compound 25 (300 mg, 0.43 mmol), followed by stirring the mixture for 24 hours at room temperature. A saturated aqueous solution of sodium bicarbonate was added, and the mixture was extracted with chloroform. The extract was washed with a 0.5 M aqueous solution of potassium bisulfate, water and a saturated aqueous solution of sodium chloride, and then dried over anhydrous sodium sulfate. The solvent was distilled off under reduced pressure, and the resulting crude product was purified by column chromatography (ethyl acetate:chloroform = 1:6) to obtain Compound 26 (173 mg, 54%) as a white foamy substance.
¹H-NMR (CDCl₃) δ: 1.21 (3H, s), 2.14 (1H, dd, J = 10, 13 Hz), 2.36 (1H, dd, J = 5, 13 Hz), 3.17-3.25 (1H, m), 3.24 (3H, s), 3.35-3.46 (1H, m), 3.68-3.75 (2H, m), 3.79 (3H, s), 4.11 (1H, brs), 4.25, 4.40 (2H, ABq, J = 6 Hz), 4.71 (2H, s), 5.50 (2H, s), 6.59 (1H, dd, J = 5, 10 Hz), 6.82-6.86 (4H, J = 9 Hz), 7.23-7.40 (14H, m), 7.84 (1H, s).

### (6) 3'-O-Benzyl-3-N-benzyloxymethyl-3'-C-methoxymethyloxymethyl-5'-0-(4,4'-dimethoxytrityl)thymidine (Compound 27)

In a stream of nitrogen, an anhydrous tetrahydrofuran solution (4.0 ml) of Compound 26 (476 mg, 0.64 mmol) was added, while cooled with ice, to an anhydrous tetrahydrofuran suspension (2.0 ml) of sodium hydride (51 mg, 1.3 mmols), followed by stirring the mixture for 1 hour at room temperature. With the system being cooled with ice, benzyl bromide (0.15 ml, 1.2 mmols) and tetrabutylammonium iodide (0.35 g, 0.96 mmol) were added, and the mixture was stirred for 19 hours at room temperature. Methanol was added, and the solvent was distilled off under reduced pressure, whereafter the residue was extracted with ethyl acetate. The organic layer was washed with water and a saturated aqueous solution of sodium chloride, and then dried over anhydrous sodium sulfate. The solvent was distilled off under reduced pressure, and the resulting crude product was purified by flash column chromatography (ethyl acetate:n-hexane = 1:2) to obtain Compound 27 (428 mg, 81%) as a white foamy substance.
¹H-NMR (CDCl₃) δ: 1.24 (3H, s), 2.10 (1H, dd, J = 9, 13 Hz), 2.69 (1H, dd, J = 5, 13 Hz), 3.21 (3H, s), 3.31-3.35 (1H, m), 3.55, 3.94 (2H, ABq, J = 11 Hz), 3.73 (1H, dd, J = 4, 11 Hz), 3.79 (6H, s), 4.04 (1H, d, J = 6 Hz), 4.29-4.31 (1H, m), 4.57 (2H, s), 4.71 (2H, s), 5.49 (2H, s), 6.53 (1H, d, J = 5, 10 Hz), 6.82-6.86 (4H, d, J = 9 Hz), 7.24-7.85 (19H, m). 7.86 (1H, s).

### (7) 3'-O-Benzyl-3-N-benzyloxymethyl-3'-C-(methoxymethyloxymethyl)thymidine (Compound 28)

(+)-10-Camphorsulfonic acid (24 mg, 0.1 mmol) was added to a tetrahydrofuran-methanol solution (2:1, 6 ml) of Compound 27 (428 mg, 0.52 mmol), and the mixture was stirred for 40 minutes at room temperature. A saturated aqueous solution of sodium bicarbonate was added, and the mixture was extracted with ethyl acetate. The organic layer was washed with water and a saturated aqueous solution of sodium chloride, and then dried over anhydrous sodium sulfate. The solvent was distilled off under reduced pressure, and the resulting crude product was purified by column chromatography (ethyl acetate:n-hexane = 2:1) to obtain Compound 28 (248 mg, 91%) as a colorless oily substance.
¹H-NMR (CDCl₃) δ: 1.94 (3H, d, J = 1 Hz), 2.24 (1H, dd, J = 9, 14 Hz), 2.59 (1H, dd, J = 6, 14 Hz), 3.00 (1H, t, J = 6 Hz), 3.40 (3H, s), 3.89-4.02 (2H, m), 3.95, 3.98 (2H, ABq, J = 11 Hz), 4.24 (1H, m), 4.61 (2H, s), 4.70 (4H, s), 5.50 (2H, s), 6.19 (1H, dd, J = 5, 9 Hz), 7.28-7.39 (10H, m), 7.60 (1H, d, J = 1 Hz).

### (8) Compound 29

A dichloromethane solution (1 ml) of Compound 28 (57 ml, 0.11 mmol) was added to a dichloromethane solution (1 ml) of Dess-Martian periodinane (91 mg, 0.22 mmol), and the mixture was stirred for 30 minutes at room temperature. A saturated aqueous solution of sodium thiosulfate and a saturated aqueous solution of sodium bicarbonate were added, and stirring was continued until the reaction mixture became transparent. The reaction mixture was extracted with dichloromethane, and the extract was washed with water and a saturated aqueous solution of sodium chloride, and then dried over anhydrous sodium sulfate. The solvent was distilled off under reduced pressure to obtain Compound 29 (64 mg) as a crude product.
¹H-NMR (CDCl₃) δ: 2.00 (3H, s), 2.00-2.08 (1H, m), 2.69 (1H, dd, J = 5, 14 Hz), 3.38 (3H, s), 3.68, 3.92 (2H, ABq, J = 11 Hz), 4.61-4.71 (7H, m), 5.50 (2H, s), 6.48 (1H, dd, J = 5, 9 Hz), 7.28-7.36 (10H, m), 7.83 (1H , d, J = 1 Hz), 9.78 (1H, s).

### (9) 3'-O-Benzyl-3-N-benzyloxymethyl-4'-C-hydroxymethyl-3'-C-(methoxymethyloxymethyl)thymidine (Compound 30)

A 37% aqueous solution (22 µl, 0.28 mmol) of formaldehyde and a 1N aqueous solution (80 µl, 0.08 mmol) of sodium hydroxide were added, while cooled with ice, to a tetrahydrofuran solution (1 ml) of Compound 29 (58 mg, 0.11 mmol) as the crude product, and the mixture was stirred for 2 hours at room temperature. Then, sodium borohydride (8 mg, 0.22 mmol) was added, while cooled with ice, and the mixture was stirred for 1.5 hours. After neutralization with a 0.5M aqueous solution of potassium bisulfate, and the system was extracted with ethyl acetate. The organic layer was washed with water and a saturated aqueous solution of sodium chloride, and then dried over anhydrous sodium sulfate. The solvent was distilled off under reduced pressure, and the resulting crude product was purified by flash column chromatography (ethyl acetate:n-hexane = 2:1) to obtain Compound 30 (25 mg, 41%) as a colorless oily substance.
¹H-NMR (CDCl₃) δ: 1.93 (3H, s), 2.35 (1H, dd, J = 9, 12Hz), 2.65 (1H, dd, J = 4, 12 Hz), 2.77 (1H, dd, J = 4, 8 Hz), 3.10 (1H, brs), 3.40 (3H, s), 3.62 (1H, t, 9 Hz), 3.91-4.08 (5H, m), 4.56 (6H, m), 5.48 (2H, s), 6.05 (1H, dd, J = 4, 8 Hz), 7.28-7.36 (10H. m), 7.56 (1H, s).

### (10) 3'-O,5'-O-Dibenzyl-3-N-benzyloxymethyl-4'-C-hydroxymethyl-3'-C-(methoxy-methyloxymethyl)thymidine (Compound 31)

In a stream of nitrogen, an anhydrous N,N-dimethylformamide solution (0.4 ml) of Compound 30 (14 mg, 0.025 mmol) was added, while cooled with ice, to an anhydrous N,N-dimethylformamide solution (0.1 ml) of sodium hydride (1.4 mg, 0.035 mmol), and the mixture was stirred for 30 minutes. Benzyl bromide (3 µl, 0.026 mmol) was added, and the mixture was stirred for 1 hour. Methanol was added, and the mixture was extracted with ethyl acetate. The organic layer was washed with water and a saturated aqueous solution of sodium chloride, and then dried over anhydrous sodium sulfate. The solvent was distilled off under reduced pressure, and the resulting crude product was purified by column chromatography (ethyl acetate:n-hexane = 1:2) to obtain Compound 31 (8 mg, 48%) as a colorless oily substance.
¹H-NMR (CDCl₃) δ: 1.64 (3H, d, J = 1 Hz), 2.11 (1H, dd, J = 10, 13 Hz), 2.67 (1H, dd, J = 5, 13 Hz), 2.80 (1H, dd, J = 3, 10 Hz), 3.34 (3H, s), 3.51 (1H, t, J = 10 Hz), 3.82 (1H, d, J = 11 Hz), 3.88 (1H , d, J = 11 Hz), 3.99-4.07 (3H, m), 4.50-4.64 (6H, m), 4.67 (2H, s), 5,46 (2H, s), 6.38 (1H, dd, J = 5, 10 Hz), 7.317.35 (15H, m), 7.80 (1H, d, J = 1 Hz).

### (11) 3'-O,5'-O-Dibenzyl-3-N-benzyloxymethyl-3'-C,4'-C-bis(hydroxymethyl)thymidine (Compound 32)

In a stream of nitrogen, a 6M aqueous solution of hydrochloric acid was added, at room temperature, to a tetrahydrofuran (1 ml) solution of Compound 31 (25 mg, 0.039 mmol), and the mixture was stirred for 30 hours at 50°C. With the system being cooled with ice, a saturated aqueous solution of sodium bicarbonate was added to the reaction mixture. The system was extracted with chloroform, the extract was washed with a saturated aqueous solution of sodium chloride, and then the organic layer was dried over anhydrous sodium sulfate. The solvent was distilled off under reduced pressure, and the resulting crude product was purified by silica gel column chromatography (chloroform:methanol = 50:1) to obtain Compound 32 (17 mg, 73%) as a colorless oily substance.
¹H-NMR (CDCl₃) δ: 1.6 (3H, s), 2.1 (1H, dd, J = 10, 13 Hz), 2.6 (1H, dd, J = 5, 13 Hz), 2.9 (1H, br), 3.5-4.1 (7H, m), 4.5-4.7 (4H, m), 4.7 (2H, s), 5.4 (2H, s), 6.4 (1H, dd, J = 5, 10 Hz), 7.2-7.4 (15H, m), 7.7 (1H, d, J = 1 Hz).

### (12) 3'-O,5'-O-Dibenzyl-3-N-benzyloxymethyl-3'-C,4'-C-[methylenebis(oxymethyl)]thymidine (Compound 33)

In a stream of nitrogen, paraformaldehyde (24 mg) and p-toluenesulfonic acid monohydrate (2 mg) were added to a 1,2-dichloroethane (3 ml) solution of Compound 32 (17 mg, 0.028 mmol) at room temperature, and the mixture was refluxed for 4 hours. With the system being cooled with ice, a saturated aqueous solution of sodium bicarbonate was added to the reaction mixture. The system was extracted with chloroform, the extract was washed with a saturated aqueous solution of sodium chloride, and then the organic layer was dried over anhydrous sodium sulfate. The solvent was distilled off under reduced pressure, and the resulting crude product was purified by silica gel column chromatography (ethyl acetate:n-hexane = 1:3, then 1:2, and then 1:1) to obtain Compound 33 (9 mg, 52%) as a colorless oily substance.
¹H-NMR (CDCl₃) δ: 1.7 (3H, s), 2.4 (1H, dd, J = 9, 13 Hz), 2.9 (1H, dd, J = 5, 13 Hz), 3.7-4.7 (12H, m), 4.9, 5.3 (2H, AB, J = 8 Hz), 5.5 (2H, s), 6.3 (1H, dd, J = 5, 9 Hz), 7.2-7.4 (15H, m), 7.8 (1H, d, J = 1 Hz).

### (13) 3'-C,4'-C-[Methylenebis(oxymethyl)]thymidine (Compound 34)

In a stream of nitrogen, 20% palladium hydroxide/carbon powder (9 mg) and cyclohexene (74 µl, 0.73 mmol) were added to an ethanol (1 ml) solution of Compound 33 (9 mg, 0.015 mmol) at room temperature, and the mixture was refluxed for 4 hours. After filtration, the solvent was distilled off under reduced pressure, and the resulting crude product was purified by PTLC (chloroform:methanol = 5:1) to obtain Compound 34 (3 mg, 65%).
¹H-NMR (CD₃OD, 500 MHz) δ: 1.87 (3H, s), 2.18 (1H, dd, J = 5.5, 13.0 Hz), 2.34 (1H, dd, J = 10.0, 13.0 Hz), 3.68, 3.77 (2H, AB, J = 11.5 Hz), 3.75-3.84 (4H, m), 4.90 (2H, s), 6.35 (1H, dd, J = 5.0, 9.0 Hz), 8.12 (1H, s).

Compound 34 can be converted into an amidite compound (Compound 36), via Compound 35, by the same method as described in Example 2, or by a publicly known method.

### [Example 4] Synthesis of nucleoside analogue (an alternative method for synthesis of Compound 33)

Compound 33 can also be synthesized in accordance with the following synthesis scheme:

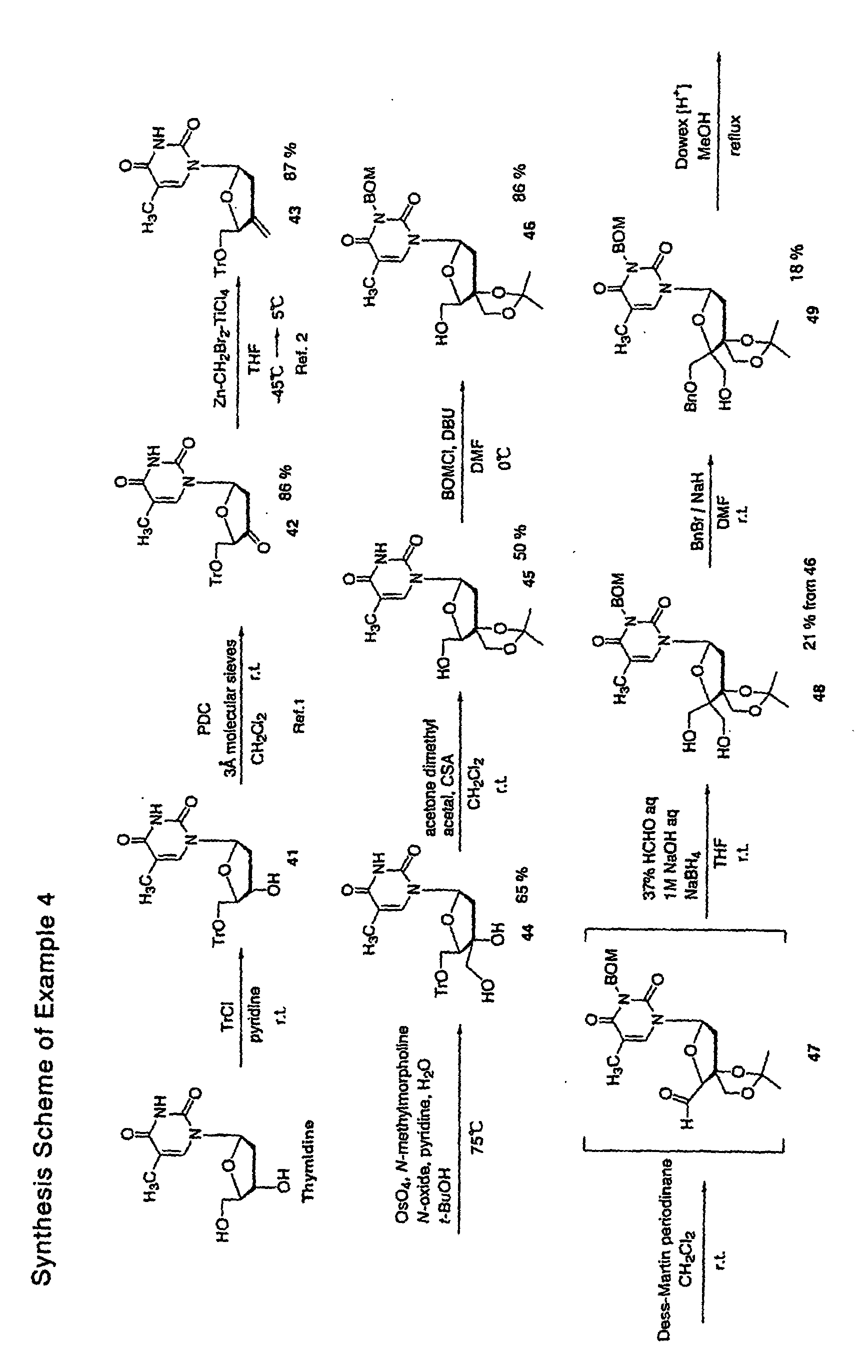

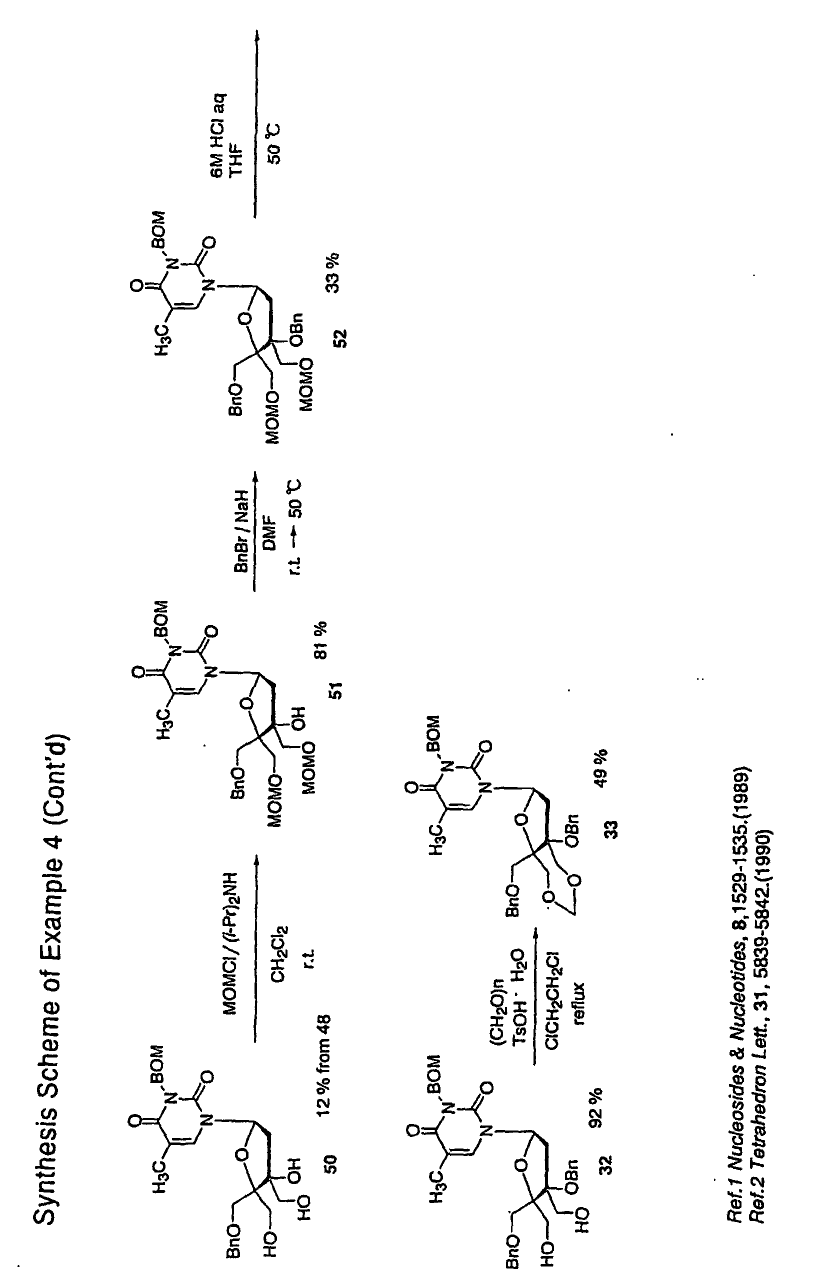

### (1) 5'-O-Tritylthymidine (Compound 41)

Synthesized in the following manner according to literature [Nucleosides & Nucleotides, 8, 1529-1535 (1989)]: In a stream of nitrogen, triphenylmethyl chloride (15 g, 53.7 mmols) was added to a pyridine (40 ml) solution of thymidine (10 g, 41.3 mmols) at room temperature, and the mixture was stirred for 21.5 hours at room temperature. A saturated aqueous solution of sodium bicarbonate was added to the reaction mixture, and the system was extracted with a methylene chloride solution. The organic layer was washed with a saturated aqueous solution of sodium chloride, and then dried over sodium sulfate. The solvent was distilled off under reduced pressure, and the resulting crude product was purified by silica gel column chromatography (ethyl acetate:n-hexane = 1:1)* to obtain Compound 41 (19.4 g, 97.1%) as a colorless acicular substance.

*Alternatively, purification can be performed by recrystallization (ethyl acetate). mp. 107-110°C.
¹H-NMR (CDCl₃) δ: 1.50 (3H, d, J = 1 Hz), 2.3-2.5 (2H, m), 3.4 (1H, dd, J = 3, 11 Hz), 3.5 (1H, dd, J = 3, 11 Hz), 4.0 (1H, dd, J = 3, 6 Hz), 4.6 (1H, dd, J = 3, 6 Hz), 6.4 (1H, dd, J = 6.8 Hz), 7.2-7.4 (15H, m), 8.5 (1H, s).

### (2) 3'-Keto-5'-O-tritylthymidine (Compound 42)

Synthesized in the following manner according to literature [Nucleosides & Nucleotides, 8, 1529-1535 (1989)]: In a stream of nitrogen, Compound 41 (4.54 g, 9.36 mmols) was added to a methylene chloride solution (36 ml) of a 3Å molecular sieve powder (5.46 g, Sigma M1885) and pyridinium dichromate (5.46 g, 14.51 mmols) at room temperature. The wall of a flask was washed with a methylene chloride solution (36 ml), and the system was stirred for 3 hours at room temperature. The reaction mixture was flowed over a glass filter filled with a 3A molecular sieve powder, and the glass filter was rinsed with methylene chloride, whereafter the solvent was distilled off under reduced pressure. Ethyl acetate was added to the resulting solid residue, and the mixture was dissolved upon exposure to ultrasonic waves. The resulting solution was flowed over a glass filter filled with a 3A molecular sieve powder, and the glass filter was rinsed with methylene chloride. Then, the solvent was distilled off under reduced pressure to obtain Compound 42 (3.87 g, 85.7%) as a yellow solid substance.
mp. 172-174°C.
¹H-NMR (CDCl₃) δ: 1.3 (3H, d, J = 1 Hz), 2.8 (1H, dd, J = 8, 19 Hz), 3.1 (1H, dd, J = 7, 19 Hz), 3.4 (1H, dd, J= 2, 11 Hz), 3.7 (1H, dd, J = 3, 10 Hz), 4.2 (unresolved dd-appears as br, s), 6.6 (1H, dd, J = 7, 8 Hz), 7.2-7.4 (15H, m), 7.6 (unresolved q-appears as d), 8.2 (1H, br, s).

### (3) 3'-Deoxy-5'-O-trityl-3'-C-methylenethymidine (Compound 43)

In a stream of nitrogen, titanium tetrachloride (21 ml) was slowly added dropwise, at -45°C, to a tetrahydrofuran (450 ml) solution of zinc powder (51.4 g) and dibromomethane (18 ml), and the mixture was stirred for 3 days at 5°C. In a stream of nitrogen, a methylene chloride (300 ml) solution of Compound 42 (17.8 g, 37.3 mmols) was slowly added dropwise to the resulting suspension, with the system being cooled with ice. The mixture was stirred for 11 hours at room temperature. A saturated aqueous solution of sodium bicarbonate was added to the reaction mixture, and the mixture was flowed over a glass filter filled with silica gel, whereafter the mother liquor was extracted with chloroform. The resulting organic layer was washed with water and a saturated aqueous solution of sodium chloride, and then dried over anhydrous sodium sulfate. The solvent was distilled off under reduced pressure to obtain Compound 43 (15.5 g, 86.6%) as a yellow solid substance.

Reference: Tetrahedron Lett. 31, 5839-5842 (1990). mp. 84-85°C.
¹H-NMR (CDCl₃) δ: 1.6 (3H, s), 2.7-2.9 (1H, m), 3.1 (1H, dd, J = 6, 15 Hz), 3.4 (2H, d, J = 3 Hz), 4.6 (1H, d, J = 2 Hz), 5.0 (1H, d, J = 1 Hz), 5.3 (1H, d, J = 2 Hz), 6.3 (1H, d,d J = 6, 10 Hz), 7.2-7.5 (15 H, m), 7.6 (1H, d, J = 1 Hz), 8.0 (1 H, s).

### (4) 3'-C-Hydroxymethyl-5'-0-tritylthymidine (Compound 44)

In a stream of nitrogen, N-methylmorpholine N-oxide (9.6 g, 82.1 mmols), pyridine (5.1 ml, 63.1 mmols), water (6.7 ml) and a t-butyl alcohol solution (0.6 ml) of 0.076 M osmium tetroxide were added to a t-butyl alcohol solution (100 ml) of Compound 43 (5.7 g, 11.9 mmols) at room temperature, followed by stirring the mixture for 8 hours at 75°C. A saturated aqueous solution of sodium thiosulfate was added to the reaction mixture, and the system was extracted with ethyl acetate. The organic layer was washed with a saturated aqueous solution of sodium chloride, and then dried over sodium sulfate, whereafter the solvent was distilled off under reduced pressure. The resulting crude product was purified by silica gel column chromatography (chloroform:methanol = 20:1) to obtain Compound 44 (4.0 g, 65.0%) as a colorless solid substance.
mp. 112-114°C.
¹H-NMR (CDCl₃) δ: 1.4 (1H, d, J = 1 Hz), 2.1 (1H, dd, J = 9, 13 Hz), 2.4 (1H, dd, J = 5, 13 Hz), 3.1 (1H, t, J = 6 Hz), 3.2 (1H, dd, J = 2, 11 Hz), 3.5-3.7 (3H, m), 4.1 (1H, dd, J = 2, 4 Hz), 6.4 (1H, dd, J = 5, 9 Hz), 7.2-7.5 (15H, m), 7.6 (1H, d, J = 1 Hz), 9.5 (1H, s).

### (5) 3'-C-(Hydroxymethyl)thymidine 3'-O,3'-C-CH₂O-Acetonide (Compound 45)

In a stream of nitrogen, acetone dimethyl acetal (1.9 ml, 15.2 mmols) and (+)-10-camphorsulfonic acid (229 mg, 0.99 mmol) were added to a methylene chloride (60 ml) solution of Compound 44 (3.9 g, 7.6 mmols) at room temperature, and the mixture was stirred for 22.5 hours at room temperature. A saturated aqueous solution of sodium bicarbonate was added, and the mixture was extracted with ethyl acetate. The organic layer was washed with a saturated aqueous solution of sodium chloride, and then dried over anhydrous sodium sulfate. The solvent was distilled off under reduced pressure, and the resulting crude product was purified by silica gel column chromatography (ethyl acetate:n-hexane = 1:3, then 1:2, and then 1:1) to obtain Compound 45 (1.2 g, 50.4%) as a colorless solid substance.
mp. 104-105°C.
¹H-NMR (CDCl₃) δ: 1.35 (3H; s), 1.40 (3H, s), 1.9 (3H, s), 2.3 (1H, dd, J = 5, 13 Hz), 2.4 (1H, dd, J = 10, 13 Hz), 3.7 (1H, d, J = 12 Hz), 3.9 (1H, d, J = 12 Hz), 4.07 (1H, d, J = 10 Hz), 4.09 (1H, s), 4.2 (1H, d, J = 10 Hz), 6.0 (1H, dd, J = 6, 9 Hz), 7.4 (1H, s), 9.5 (1H, br).

### (6) 3-N-Benzyloxymethyl-3'-C-(hydroxymethyl)thymidine 3'-O,3'-C-CH₂O-Acetonide (Compound 46)

In a stream of nitrogen, 1,8-diazabicyclo[5.4.0]-undeca-7-ene (0.73 ml, 4.9 mmols) and benzchloromethyl ether (0.68 ml, 4.9 mmols) were added, while cooled with ice, to a dimethylformamide (5 ml) solution of Compound 45 (1.27 g, 4.07 mmols), followed by stirring the mixture for 5 hours while cooling it with ice. Water was added to the reaction mixture, and the system was extracted with ethyl acetate. The extract was washed with a saturated aqueous solution of sodium chloride, and then the organic layer was dried over sodium sulfate. The solvent was distilled off under reduced pressure, and the resulting crude product was purified by column chromatography (ethyl acetate:n-hexane = 1:1) to obtain Compound 46 (1.6 g, 86%) as a white powdery substance.
mp. 196-198°C.
¹H-NMR (CDCl₃) δ: 1.38 (3H, s), 1.44 (3H, s), 1.9 (3H, s), 2.3 (1H, dd, J = 6, 13 Hz), 2.4 (1H, dd, J = 9, 13 Hz), 3.2 (1H, dd, J = 3, 7 Hz), 3.8 (1H, ddd, J = 2, 6, 12 Hz), 3.9-4.0 (1H, m), 4.10, 4.2 (2H, AB, J = 10 Hz), 4.13 (1H, s), 4.7 (2H, s), 5.5 (2H, s), 6.0 (1H, dd, J = 6, 9 Hz), 7.2-7.4 (5H, m), 8.0 (1H, s).

### (7) 3-N-Benzyloxymethyl-3'-C,4'-C-bis(hydroxymethyl)thymidine 3'-O,3'-C-CH₂O-Acetonide (Compound 48)

In a stream of nitrogen, a methylene chloride solution (10 ml) of Compound 46 (1.00 g, 2.31 mmols) was added to a methylene chloride (10 ml) solution of the Dess-Martin reacting agent (2.23 g, 5.27 mmols) at room temperature, and the mixture was stirred for 5 hours at room temperature. A saturated aqueous solution of sodium thiosulfate and a saturated aqueous solution of sodium bicarbonate were added, and the system was extracted with methylene chloride. The organic layer was washed with a saturated aqueous solution of sodium chloride, and then dried over anhydrous sodium sulfate. The solvent was distilled off under reduced pressure, and a 37% aqueous solution (4.5 ml) of formaldehyde and a 1M aqueous solution (25 ml) of sodium hydroxide were added to a tetrahydrofuran (70 ml) solution of the resulting crude product (Compound 47) at room temperature in a stream of nitrogen. After stirring was performed for 16 hours at room temperature, sodium borohydride (420 mg, 0.11 mmol) was added, and the mixture was stirred for 4 hours. Dowex [H⁺] was added to the reaction mixture to neutralize it. After filtration through a fluted filter, the system was washed with methanol. Then, the solvent was distilled off under reduced pressure, and the residue was extracted with chloroform. The organic layer was washed with a saturated aqueous solution of sodium chloride, and then dried over anhydrous sodium sulfate. The solvent was distilled off under reduced pressure, and the resulting crude product was purified by silica gel column chromatography (chloroform:methanol = 40:1) to obtain Compound 48 (221 mg, 21%).
¹H-NMR (CDCl₃) δ: 1.4 (3H, s), 1.5 (3H, s), 1.9 (3H, s), 2.4 (1H, dd, J = 5, 13 Hz), 2.7 (1H, dd, J = 10, 13 Hz), 3.1 (1H, br), 3.6-3.9 (4H, m), 4.2 (1H, d, J = 10 Hz), 4.3 (1H, d, J = 10 Hz), 4.7 (2H, s), 5.5 (2H, s), 6.0 (1H, dd, J = 5, 10 Hz), 7.3-7.4 (6H, m).

### (8) 5'-O-Benzyl-3-N-benzyloxymethyl-3'-C,4'-C-bis(hydroxymethyl)thymidine 3'-O,3'-C-CH₂O-Acetonide (Compound 49)

In a stream of nitrogen and in an ice-cooled environment, Compound 48 (489 mg, 1.06 mmols) was added to a dimethylformamide (2 ml) solution of 60% sodium hydride (55 mg, 1.4 mmols), and the mixture was stirred for 15 minutes. Then, benzyl bromide (0.15 ml, 1.3 mmols) was added, and the mixture was stirred for 7 hours at room temperature. Water was added to the reaction mixture, and the system was extracted with ethyl acetate. The extract was washed with a saturated aqueous solution of sodium chloride, and then the organic layer was dried over anhydrous sodium sulfate. The solvent was distilled off under reduced pressure, and the resulting crude product was purified by silica gel column chromatography (ethyl acetate:n-hexane = 1:2) to obtain Compound 49 as a mixture with its regioisomer (123 mg, (Compound 49:its regioisomer = 5:1)).
¹H-NMR (CDCl₃) δ: 1.4 (3H, s), 1.5 (3H, s), 1.7 (3H, s), 2.2 (1H, m), 2.5 (1H, dd, J = 5, 13 Hz), 3.62 (1H, dd, J = 8, 12 Hz), 3.65 (1H, dd, J = 11 Hz), 3.7 (1H, dd, J = 5, 12 Hz), 3.8 (1H, d, J = 11 Hz), 4.0 (1H, d, J = 10 Hz), 4.2 (1H, d, J = 10 Hz), 4.6 (2H, s), 4.7 (2H, s), 5.5 (2H, s), 6.4 (1H, dd, J = 5, 10 Hz), 7.2-7.4 (10H, m), 7.6 (1H, d, J = 1 Hz).

### (9) 5'-O-Benzyl-3-N-benzyloxymethyl-3'-C,4'-C-bis(hydroxymethyl)thymidine (Compound 50)

In a stream of nitrogen and at room temperature, Dowex [H⁺] (250 mg) was added to a methanol solution of the mixture (123 mg) obtained in the previous step, and the system was stirred for 8 hours. The reaction mixture was filtered through a fluted filter, and concentrated thereby. The residue was purified by silica gel column chromatography (ethyl acetate:n-hexane = 2:1) to obtain Compound 50 (67 mg, 12% calculated from Compound 49) as a colorless oily substance.
¹H-NMR (CDCl₃) δ: 1.5 (3H, s), 2.0 (1H, br), 2.2-2.3 (2H, m), 3.6 (1H, d, J = 10 Hz), 3.7 (1H, d, J = 10 Hz), 3.5-3.9 (6H, m), 4.5 (1H, d, J = 11 Hz), 4.61 (1H, d, J = 11 Hz), 4.64 (2H, s), 5.4 (2H, s), 6.5 (1H, dd, J = 6, 9 Hz), 7.2-7.4 (10H, m), 7.6 (1H, s).

### (10) 5'-O-Benzyl-3-N-benzyloxymethyl-3'-C,4'-C-bis(methoxymethyloxymethyl)thymidine (Compound 51)

In a stream of nitrogen and at room temperature, diisopropylethylamine (0.24 ml, 1.4 mmols) and methoxymethyl chloride (0.12 ml, 1.6 mmols) were added to a methylene chloride (1.5 ml) solution of Compound 50 (41 mg, 0.08 mmol), and the mixture was stirred for 24.5 hours at room temperature. A saturated aqueous solution of sodium bicarbonate was added to the reaction mixture, and the system was extracted with ethyl acetate. The extract was washed with a saturated aqueous solution of sodium chloride, and then the organic layer was dried over anhydrous sodium sulfate. The solvent was distilled off under reduced pressure, and the resulting crude product was purified by silica gel column chromatography (ethyl acetate:n-hexane = 1:2, then 1:1) to obtain Compound 51 (38 mg, 81%) as a colorless oily substance.
¹H-NMR (CDCl₃) δ: 1.6 (3H, s), 2.3 (2H, m), 3.25 (1H, s), 3.35 (3H, s), 3.38 (3H, s), 3.6 (1H, d, J = 11 Hz), 3.7 (1H, d, J = 10 Hz), 3.80 (1H, d, J = 10 Hz), 3.83 (1H, d, J = 10 Hz), 3.87 (1H, d, J = 10 Hz), 3.89 (1H, d, J = 11 Hz), 4.58 (2H, s), 4.63 (2H, d, J = 1 Hz), 4.66 (4H, s), 5.5 (2H, s), 6.5 (1H, dd, J = 6, 9 Hz), 7.2-7.4 (10H, m), 7.7 (1H, d, J = 1 Hz).

### (11) 3'-O,5'-O-Dibenzyl-3-N-benzyloxymethyl-3'-C,4'-C-bis(methoxymethoxymethyl)thymidine (Compound 52)

In a stream of nitrogen and in an ice-cooled environment, Compound 51 (38 mg, 0.065 mmol) was added to a dimethylformamide (1.0 ml) solution of 60% sodium hydride (5.2 mg, 0.13 mmol), and the mixture was stirred for 15 minutes. Then, benzyl bromide (15 µl, 0.13 mmol) was added, and the mixture was stirred for 5 hours at room temperature and then stirred for 3 hours at 50°C. Water was added to the reaction mixture, and the system was extracted with ethyl acetate. The extract was washed with water and a saturated aqueous solution of sodium chloride, and then the organic layer was dried over anhydrous sodium sulfate. The solvent was distilled off under reduced pressure, and the resulting crude product was purified by silica gel column chromatography (ethyl acetate:n-hexane = 1:2, then 1:1) to obtain Compound 52 (15 mg, 33%) as a colorless oily substance.
¹H-NMR (CDCl₃) δ: 1.6 (3H, s), 2.2 (1H, dd, J = 10, 13 Hz), 2.7 (1H, dd, J = 5, 13 Hz), 3.31 (3H, s), 3.33 (3H, s), 3.8-4.1 (6H, m), 4.5 (2H, d, J= 2 Hz), 4.6 (6H, s, br), 4.7 (2H, s), 5.4 (2H, s), 6.5 (1H, dd, J = 5, 10 Hz), 7.2-7.4 (15H, m), 7.8 (1H, d, J = 1 Hz).

### (12) 3'-O,5'-O-Dibenzyl-3-N-benzyloxymethyl-3'-C,4'-C-bis(hydroxymethyl)thymidine (Compound 32)

In a stream of nitrogen, a 6M aqueous solution of hydrochloric acid was added to a tetrahydrofuran (0.5 ml) solution of Compound 52 (15 mg, 0.022 mmol) at room temperature, and the mixture was stirred for 10 hours at 50°C. A saturated aqueous solution of sodium bicarbonate was added to the reaction mixture, and the system was extracted with chloroform. The extract was washed with a saturated aqueous solution of sodium chloride, and then the organic layer was dried over anhydrous sodium sulfate. The solvent was distilled off under reduced pressure, and the resulting crude product was purified by silica gel column chromatography (chloroform:methanol = 50:1) to obtain Compound 32 (12 mg, 92%) as a colorless oily substance.
¹H-NMR (CDCl₃) δ: 1.6 (3H, s), 2.1 (1H, dd, J = 10, 13 Hz), 2.6 (1H, dd, J = 5, 13 Hz), 2.9 (1H, br), 3.5-4.1 (7H, m), 4.5-4.7 (4H, m), 4.7 (2H, s), 5.4 (2H, s), 6.4 (1H, dd, J = 5, 10 Hz), 7.2-7.4 (15H, m), 7.7 (1H, d, J = 1 Hz).

### (13) 3'-0,5'-O-Dibenzyl-3-N-benzyloxyrnethyl-3'-C,4'-C-[methylenebis(oxymethyl)]thymidine (Compound 33)

In a stream of nitrogen, paraformaldehyde (24 mg) and p-toluenesulfonic acid monohydrate (2 mg) were added to a 1,2-dichloromethane (2 ml) solution of Compound 32 (12 mg, 0.020 mmol) at room temperature, and the mixture was refluxed for 4.5 hours. Ethyl acetate was added to the reaction mixture, and the resulting mixture was washed with a saturated aqueous solution of sodium bicarbonate and a saturated aqueous solution of sodium chloride. Then, the organic layer was dried over anhydrous sodium sulfate. The solvent was distilled off under reduced pressure, and the resulting crude product was purified by silica gel column chromatography (ethyl acetate:n-hexane = 1:3) to obtain Compound 33 (6 mg, 49%) as a colorless oily substance.
¹H-NMR (CDCl₃) δ: 1.7 (3H, s), 2.4 (1H, dd, J = 9, 13 Hz), 2.9 (1H, dd, J = 5, 13 Hz), 3.7-4.7 (12H, m), 4.9, 5.3 (2H, AB, J = 8 Hz), 5.5 (2H, s), 6.3 (1H, dd, J = 5, 9 Hz), 7.2-7.4 (15H, m), 7.8 (1H, d, J = 1 Hz).

### [Example 5] Synthesis of trans-3',4'-arabinoBNA-thymidine

The desired Compound 68 was synthesized in accordance with the following synthesis scheme:

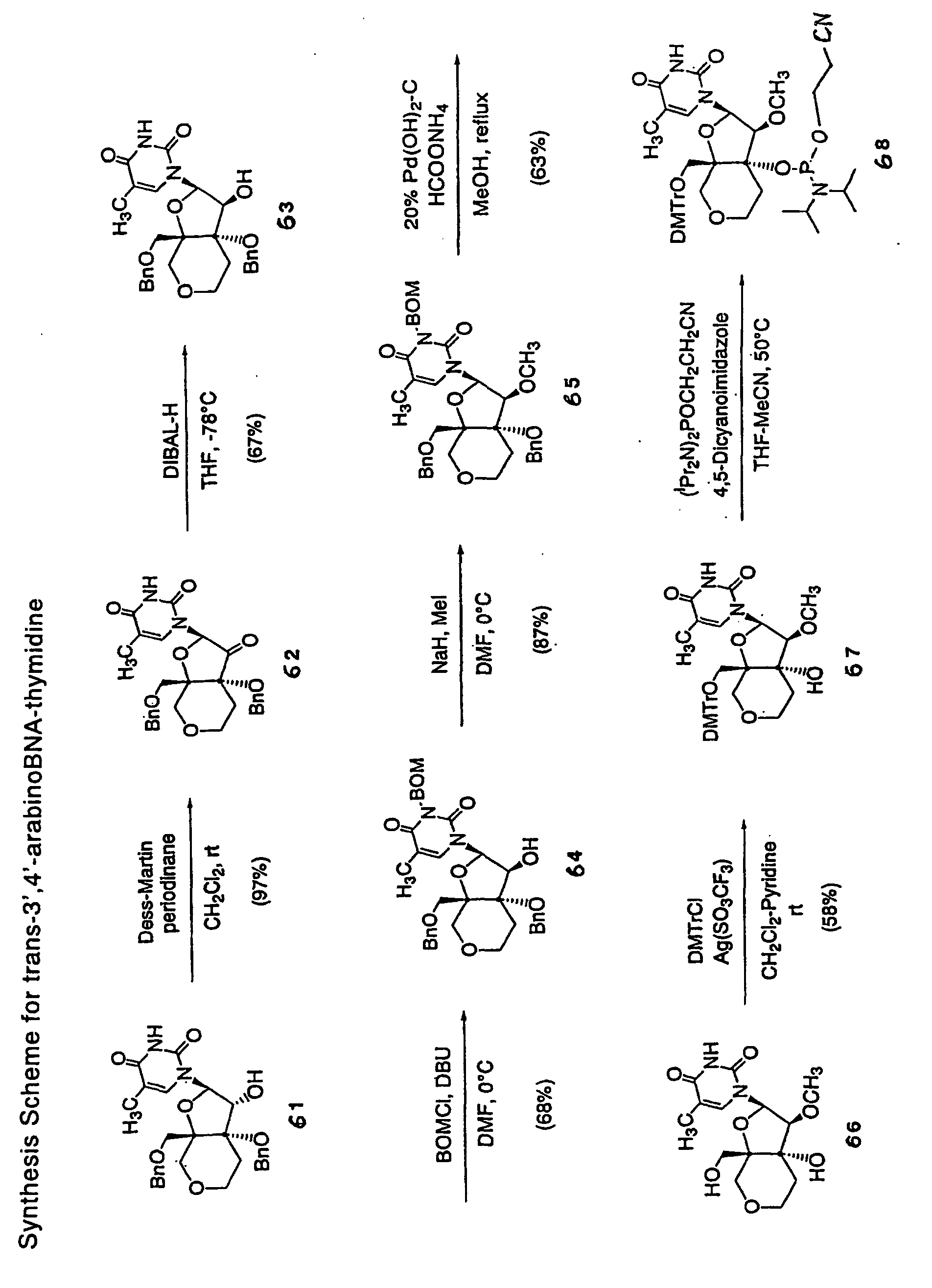

### (1) Synthesis of Compound 62

A dichloromethane solution (15 ml) of Compound 61 (979 mg, 1.98 mmols) was added to a dichloromethane suspension (5 ml).of Dess-Martian periodinane (1.26 g, 2.97 mmols), and the mixture was stirred for 30 minutes. A 2:1 solution of a saturated aqueous solution of sodium bicarbonate and a saturated aqueous solution of sodium thiosulfate was added, and the mixture was extracted with dichloromethane. The organic layer was washed with a saturated aqueous solution of sodium thiosulfate, water and a saturated aqueous solution of sodium chloride, and then dried over anhydrous sodium sulfate. The solvent was distilled off under reduced pressure, and the resulting crude product was purified by silica gel column chromatography (ethyl acetate:n-hexane = 1:1) to obtain Compound 62 (945 mg, 97%) as a white solid substance. mp. 86-88°C. ¹H-NMR (CDCl₃) δ: 1.49 (3H, s), 1.93-2.05 (1H, m), 2.27-2.32 (1H, m), 3.50, 4.23 (2H, AB, J = 11 Hz), 3.75-3.82 (1H, m), 3.82-3.91 (1H, m), 3.83, 4.52 (2H, AB, J = 11 Hz), 4.34 (2H, t, J = 10 Hz), 4.52 (2H, s), 6.51 (1H, s), 7.21-7.40 (10H, m), 7.45 (1H, s), 8.52 (1H, broad).

### (2) Synthesis of Compound 63

In a stream of nitrogen, diisobutylaluminum hydride (0.532 ml, 0.532 mmol) was added, at -78°C, to an anhydrous tetrahydrofuran solution of Compound 62 (65.4 mg, 0.133 mmol), and the mixture was stirred for 5 hours. After a saturated aqueous solution of ammonium chloride was added, the mixture was filtered through Celite, and extracted with ethyl acetate. The organic layer was washed with water and a saturated aqueous solution of sodium chloride, and then dried over anhydrous sodium sulfate. The solvent was distilled off under reduced pressure, and the resulting crude product was purified by silica gel column chromatography (chloroform:methanol = 40:1) to obtain Compound 63 (44 mg, 67%) as a white solid substance.
mp. 133-135°C. ¹H-NMR (acetone-d₆) δ: 1.70 (3H, d, J = 1 Hz), 1.96-2.06 (1H, m), 2.23-2.29 (1H, m), 3.68-3.77 (1H, dt, J = 3, 11 Hz), 3.82-3.87 (1H, dd, J = 4, 11 Hz), 4.05, 4.09 (2H, AB, J = 9 Hz), 4.14, 4.25 (2H, AB, J = 10 Hz), 4.56, 4.70 (2H, AB, J = 11 Hz), 4.58-4.72 (2H, m), 4.88 (1H, broad t, J = 5 Hz), 5.25 (1H, d, J = 6 Hz), 6.10 (1H, d, J = 4 Hz), 7.26-7.45 (10H, m), 7.90 (1H, d, J = 1 Hz), 10.05 (1H, broad).

### (3) Synthesis of Compound 64

In a stream of nitrogen, 1,8-diazabicyclo[4.3.0]-non-7-ene (0.062 ml, 0.415 mmol) and benzylchloromethyl ether (0.057 ml, 0.415 mmol) were added, while cooled with ice, to a dimethylformamide solution of Compound 63 (100.7 mg, 0.207 mmol), followed by stirring the mixture for 3 hours. After methanol was added, the solvent was distilled off under reduced pressure, and the residue was extracted with dichloromethane. The organic layer was washed with water and a saturated aqueous solution of sodium chloride, and then dried over anhydrous sodium sulfate. The solvent was distilled off under reduced pressure, and the resulting crude product was purified by silica gel column chromatography (ethyl acetate:n-hexane = 2:3) to obtain Compound 64 (89.2 mg, 68%) as a white solid substance.
mp. 72-74°C. ¹H-NMR (acetone-d₆) δ: 1.74 (3H, d, J = 2 Hz), 2.00-2.07 (1H, m), 2.24-2.28 (1H, m), 3.74 (1H, dt, J = 4, 11 Hz), 3.85 (1H, dd, J = 5, 11 Hz), 4.07, 4.10 (2H, AB, J = 10 Hz), 4.12, 4.21 (2H, AB, J = 10 Hz), 4.60, 4.69 (2H, AB, J = 12 Hz), 4.61, 4.67 (2H, AB, J = 12 Hz), 4.65 (2H, s), 4.94 (1H, dd, J = 5, 6 Hz), 5.19 (1H, d, J = 6 Hz), 5.40, 5.46 (2H, AB, J = 9 Hz), 6.11 (1H, d, J = 5 Hz), 7.24-7.45 (15H, m), 7.95 (1H, d, J = 2 Hz).

### (4) Synthesis of Compound 65

In a stream of nitrogen, a dimethylformamide solution of Compound 64 (87 mg, 0.142 mmol) was added to a dimethylformamide suspension of sodium hydride (10.8 mg, 0.283 mmol), with the system being cooled with ice, followed by stirring the mixture for 30 minutes. Then, iodomethane (0.044 ml, 0.708 mmol) was added, while cooled with ice, and the mixture was stirred for 30 minutes. After methanol was added, the solvent was distilled off under reduced pressure, and the residue was extracted with ethyl acetate. The organic layer was washed with water and a saturated aqueous solution of sodium chloride, and then dried over anhydrous sodium sulfate. The solvent was distilled off under reduced pressure, and the resulting crude product was purified by silica gel column chromatography (ethyl acetate:n-hexane = 2:3) to obtain Compound 65 (77.5 mg, 87%) as a white solid substance.
mp. 86-88°C. ¹H-NMR (acetone-d₆) δ: 1.72 (3H, d, J = 1 Hz), 2.00-2.07 (1H, m), 2.38-2.44 (1H, m), 3.39 (3H, s), 3.73 (1H, m), 3.86 (1H, m), 3.93, 4.10 (2H, AB, J = 10 Hz), 4.09 (2H, s), 4.46 (1H, d, J = 5 Hz), 4.57, 4.67 (2H, AB, J = 12 Hz), 4.67 (2H, s), 4.72 (2H, s), 5.45, 5.49 (2H, AB, J = 9 Hz), 6.05 (1H, d, J = 5 Hz), 7.24-7.48 (15H, m), 7.91 (1H, d, J = 1 Hz).
¹H-NMR (CDCl₃) δ: 1.80 (3H, d, J = 1 Hz), 1.90-2.02 (1H, m), 2.13-2.19 (1H, m), 3.35 (3H, s), 3.71 (1H, m), 3.87 (1H, m), 3.82, 4.01 (2H, AB, J = 10 Hz), 4.18 (2H, AB, J = 9 Hz), 4.37 (1H, d, J = 5 Hz), 4.53 (2H, s), 4.54, 4.65 (2H, AB, J = 12 Hz), 4.74 (2H, s), 5.51 (2H, s), 6.06 (1H, d, J = 5 Hz), 7.24-7.39 (15H, m), 7.91 (1H, d, J = 1 Hz).

### (5) Synthesis of Compound 66

20% Palladium hydroxide-carbon powder (48 mg) and ammonium formate (4.80 g, 7.6 mmols) were added to a methanol solution of Compound 65 (95.6 mg, 0.152 mmol), and the mixture was refluxed for 7.5 hours. After filtration through a fluted filter, the solvent was distilled off. The resulting crude product was purified by silica gel column chromatography (chloroform:methanol = 20:1) to obtain Compound 6 (31.6 mg, 63%).
¹H-NMR (CD₃OD) δ: 1.74-1.88 (1H, m), 1.88 (3H, d, J = 1 Hz), 1.97-2.09 (1H, m), 3.32 (3H, s), 3.77-3.83 (1H, m), 3.86-3.90 (1H, m), 3.93, 4.07 (2H, AB, J = 11 Hz), 3.95 (1H, d, J = 5 Hz), 3.98 (2H, s), 6.13 (1H, d, J = 5 Hz), 8.07 (1H, d, J = 1 Hz).

### (6) Synthesis of Compound 67

In a stream of nitrogen, an anhydrous tetrahydrofuran solution of 4,4'-dimethoxytrityl chloride (628 mg, 1.85 mmols) was added to an anhydrous tetrahydrofuran solution of silver trifluoromethanesulfonate (476 mg, 1.85 mmols) at room temperature, and the mixture was stirred. The supernatant was added to a pyridine-dichloromethane (1:1) solution of Compound 66 (30.4 mg, 0.0927 mmol) at room temperature in a stream of nitrogen, and the mixture was stirred for 8 hours. After a saturated aqueous solution of sodium bicarbonate was added, the mixture was extracted with ethyl acetate. The organic layer was washed with water and a saturated aqueous solution of sodium chloride, and then dried over anhydrous sodium sulfate. The solvent was distilled off under reduced pressure, and the resulting crude product was purified by silica gel column chromatography (ethyl acetate:n-hexane = 1:1) to obtain Compound 67 (34 mg, 58%) as a white solid substance.
mp. 270-272°C. ¹H-NMR (CDCl₃) δ: 1.44 (3H, d, J = 1 Hz), 1.64-1.69 (1H, m), 1.70 (3H, s), 1.76-1.88 (1H, m), 2.81 (1H, broad), 3.14 (3H, s), 3.64 (1H, m), 3.70 (1H, m), 3.76, 4.09 (2H, AB, J = 14 Hz), 3.77 (6H, s), 3.84-3.94 (2H, m), 6.05 (1H, d, J = 4 Hz), 6.77-6.82 (4H, m), 7.21-7.42 (9H, m), 7.94 (1H, d, J= 1 Hz), 9.25 (1H, broad).

### (7) Synthesis of Compound 68

In a stream of nitrogen, 2-cyanoethyl N,N,N',N'-tetraisopropylphosphorodiamidite (0.124 ml, 0.39 mmol) and 4,5-dicyanoimidazole (13 mg, 0.11 mmol) were added to an anhydrous THF-MeCN (2.0 ml, 1:1) solution of Compound 67 (34 mg, 0.05 mmol) at room temperature, and the mixture was stirred for 6.5 hours at 50°C. After a saturated aqueous solution of sodium bicarbonate was added, the mixture was extracted with ethyl acetate. The organic layer was washed with water and a saturated aqueous solution of sodium chloride, and then dried over anhydrous sodium sulfate. The solvent was distilled off under reduced pressure, and the resulting crude product was purified by silica gel column chromatography (ethyl acetate:n-hexane = 2:3) to obtain Compound 68 as a white solid substance.
mp. 75°C-77°C. ³¹P-NMR (CDCl₃) δ: 142.7, 141.4.

### [INDUSTRIAL APPLICABILITY]

The desired compounds of the present invention are nucleic acid derivatives with a modified sugar portion of nucleic acid, which are nucleotide analogues containing nucleoside analogues having the conformation of the sugar portion fixed in S-form; and which are useful as materials for various physiologically active or bioactive substances and pharmaceuticals, as functional materials for RNAi (Nature, Vol. 411, 494-498, 2001) and decoy double-stranded oligonucleotides, as functional materials for DNA chips and molecular beacons targeting single-stranded nucleic acids such as cDNA, as functional materials for uses in various antisense methods (including ribozymes and DNAzymes) or antigene methods, and as materials for high sensitivity analysis of in vivo trace components by combination with fluorescent or luminescent substances.

Since oligonucleotide analogues prepared from the nucleoside analogues of the present invention resemble DNA in stereostructure, they can be expected to provide antigenes having high affinity for DNA.

## Claims

1. A compound of the following general formula (1) and a salt thereof: 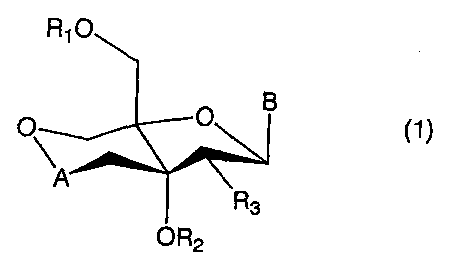 where A represents an alkylene group having 1 to 2 carbon atoms, or -CH₂-O- (where an oxygen atom is linked to a methylene group at a 3'-position),
B represents an aromatic heterocyclic group or an aromatic hydrocarbon ring group which may have a substituent,
R₁ and R₂ are identical or different, and each represent a hydrogen atom, a protective group for a hydroxyl group for synthesis of nucleic acid, an alkyl group, an alkenyl group, a cycloalkyl group, an aryl group, an aralkyl group, an acyl group, a silyl group, a phosphate group, a phosphate group protected with a protective group for synthesis of nucleic acid, or -P(R₄)R₅ [where R₄ and R₅ are identical or different, and each represent a hydroxyl group, a hydroxyl group protected with a protective group for synthesis of nucleic acid, a mercapto group, a mercapto group protected with a protective group for synthesis of nucleic acid, an amino group, an alkoxy group having 1 to 5 carbon atoms, an alkylthio group having 1 to 5 carbon atoms, a cyanoalkoxy group having 1 to 6 carbon atoms, or an amino group substituted by an alkyl group having 1 to 5 carbon atoms], and
R₃ represents a hydrogen atom, a halogen atom, a hydroxyl group, a hydroxyl group protected with a protective group for synthesis of nucleic acid, a mercapto group, a mercapto group protected with a protective group for synthesis of nucleic acid, an amino group, an amino group protected with a protective group for synthesis of nucleic acid, an alkoxy group having 1 to 5 carbon atoms, an alkylthio group having 1 to 5 carbon atoms, a cyanoalkoxy group having 1 to 6 carbon atoms, or an amino group substituted by an alkyl group having 1 to 5 carbon atoms.

2. The compound and salt thereof according to claim 1, wherein R₁ represents a hydrogen atom, an aliphatic acyl group, an aromatic acyl group, a methyl group substituted by 1 to 3 aryl groups, a methyl group substituted by 1 to 3 aryl groups, with aryl rings being substituted by a lower alkyl, lower alkoxy, halogen or cyano group, or a silyl group.

3. The compound and salt thereof according to claim 1, wherein R₁ represents a hydrogen atom, an acetyl group, a benzoyl group, a benzyl group, a p-methoxybenzyl group, a dimethoxytrityl group, a monomethoxytrityl group, or a tert-butyldiphenylsilyl group.

4. The compound and salt thereof according to any one of claim 1 to 3, wherein R₂ represents a hydrogen atom, an aliphatic acyl group, an aromatic acyl group, a methyl group substituted by 1 to 3 aryl groups, a methyl group substituted by 1 to 3 aryl groups, with aryl rings being substituted by a lower alkyl, lower alkoxy, halogen or cyano group, a silyl group, a phosphoroamidite group, a phosphonyl group, a phosphate group, or a phosphate group protected with a protective group for synthesis of nucleic acid.

5. The compound and salt thereof according to any one of claims 1 to 3, wherein R₂ represents a hydrogen atom, an acetyl group, a benzoyl group, a benzyl group, a p-methoxybenzyl group, a tert-butyldiphenylsilyl group, - P(OC₂H₄CN)(N(i-Pr)₂), -P(OCH₃)(N(i-Pr)₂), a phosphonyl group, or a 2-chlorophenyl- or 4-chlorophenylphosphate group.

6. The compound and salt thereof according to any one of claims 1 to 5, wherein R₃ represents a hydrogen atom, a halogen atom, a hydroxyl group, a hydroxyl group protected with a protective group for synthesis of nucleic acid, or an alkoxy group having 1 to 5 carbon atoms.

7. The compound and salt thereof according to any one of claims 1 to 6, wherein A represents a methylene group.

8. The compound and salt thereof according to any one of claims 1 to 7, wherein B represents a purin-9-yl group, a 2-oxo-pyrimidin-1-yl group, or a purin-9-yl group or a 2-oxo-pyrimidin-1-yl group which has a substituent selected from the following α group:
α group: A hydroxyl group, a hydroxyl group protected with a protective group for synthesis of nucleic acid, an alkoxy group having 1 to 5 carbon atoms, a mercapto group, a mercapto group protected with a protective group for synthesis of nucleic acid, an alkylthio group having 1 to 5 carbon atoms, an amino group, an amino group protected with a protective group for synthesis of nucleic acid, an amino group substituted by an alkyl group having 1 to 5 carbon atoms, an alkyl group having 1 to 5 carbon atoms, and a halogen atom.

9. The compound and salt thereof according to any one of claims 1 to 8, wherein B represents a 6-aminopurin-9-yl (i.e. adeninyl) group, a 6-aminopurin-9-yl group having an amino group protected with a protective group for synthesis of nucleic acid, a 2,6-diaminopurin-9-yl group, a 2-amino-6-chloropurin-9-yl group, a 2-amino-6-chloropurin-9-yl group having an amino group protected with a protective group for synthesis of nucleic acid, a 2-amino-6-fluoropurin-9-yl group, a 2-amino-6-fluoropurin-9-yl group having an amino group protected with a protective group for synthesis of nucleic acid, a 2-amino-6-bromopurin-9-yl group, a 2-amino-6-bromopurin-9-yl group having an amino group protected with a protective group for synthesis of nucleic acid, a 2-amino-6-hydroxypurin-9-yl (i.e. guaninyl) group, a 2-amino-6-hydroxypurin-9-yl group having an amino group protected with a protective group for synthesis of nucleic acid, a 6-amino-2-methoxypurin-9-yl group, a 6-amino-2-chloropurin-9-yl group, a 6-amino-2-fluoropurin-9-yl group, a 2,6-dimethoxypurin-9-yl group, a 2,6-dichloropurin-9-yl group, a 6-mercaptopurin-9-yl group, a 2-oxo-4-amino-1,2-dihydropyrimidin-1-yl (i.e. cytosinyl) group, a 2-oxo-4-amino-1,2-dihydropyrimidin-1-yl group having an amino group protected with a protective group for synthesis of nucleic acid, a 2-oxo-4-amino-5-fluoro-1,2-dihydropyrimidin-1-yl group, a 2-oxo-4-amino-5-fluoro-1,2-dihydropyrimidin-1-yl group having an amino group protected with a protective group for synthesis of nucleic acid, a 4-amino-2-oxo-5-chloro-1,2-dihydropyrimidin-1-yl group, a 2-oxo-4-methoxy-1,2-dihydropyrimidin-1-yl group, a 2-oxo-4-mercapto-1,2-dihydropyrimidin-1-yl group, a 2-oxo-4-hydroxy-1,2-dihydropyrimidin-1-yl (i.e. uracinyl) group, a 2-oxo-4-hydroxy-5-methyl-1,2-dihydropyrimidin-1-yl (i.e. thyminyl) group, a 4-amino-5-methyl-2-oxo-1,2-dihydropyrimidin-1-yl (i.e. 5-methylcytosinyl) group, or a 4-amino-5-methyl-2-oxo-1,2-dihydropyrimidin-1-yl group having an amino group protected with a protective group for synthesis of nucleic acid.

10. An oligonucleotide analogue containing one or more units having a structure represented by the following general formula (2), or a pharmacologically acceptable salt of said oligonucleotide analogue, provided that if said oligonucleotide analogue or salt thereof contains two or more units having one or more of said structures, B is identical or different among said structures 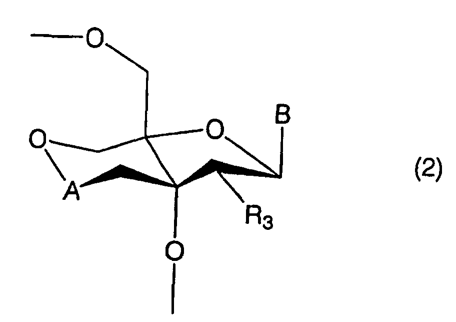 where A represents an alkylene group having 1 to 2 carbon atoms, or -CH₂-O- (where an oxygen atom is linked to a methylene group at a 3'-position),
B represents an aromatic heterocyclic group or an aromatic hydrocarbon ring group which may have a substituent, and
R₃ represents a hydrogen atom, a halogen atom, a hydroxyl group, a hydroxyl group protected with a protective group for synthesis of nucleic acid, a mercapto group, a mercapto group protected with a protective group for synthesis of nucleic acid, an amino group, an amino group protected with a protective group for synthesis of nucleic acid, an alkoxy group having 1 to 5 carbon atoms, an alkylthio group having 1 to 5 carbon atoms, a cyanoalkoxy group having 1 to 6 carbon atoms, or an amino group substituted by an alkyl group having 1 to 5 carbon atoms.

11. The oligonucleotide analogue or pharmacologically acceptable salt thereof according to claim 10, wherein R₃ represents a hydrogen atom, a halogen atom, a hydroxyl group, a hydroxyl group protected with a protective group for synthesis of nucleic acid, or an alkoxy group having 1 to 5 carbon atoms.

12. The oligonucleotide analogue or pharmacologically acceptable salt thereof according to claim 10 or 11, wherein A represents a methylene group.

13. The oligonucleotide analogue or pharmacologically acceptable salt thereof according to any one of claims 10 to 12, wherein B represents a purin-9-yl group, a 2-oxo-pyrimidin-1-yl group, or a purin-9-yl group or a 2-oxo-pyrimidin-1-yl group which has a substituent selected from the following α group:
α group: A hydroxyl group, a hydroxyl group protected with a protective group for synthesis of nucleic acid, an alkoxy group having 1 to 5 carbon atoms, a mercapto group, a mercapto group protected with a protective group for synthesis of nucleic acid, an alkylthio group having 1 to 5 carbon atoms, an amino group, an amino group protected with a protective group for synthesis of nucleic acid, an amino group substituted by an alkyl group having 1 to 5 carbon atoms, an alkyl group having 1 to 5 carbon atoms, and a halogen atom.

14. The oligonucleotide analogue or pharmacologically acceptable salt thereof according to any one of claims 10 to 13, wherein B represents a 6-aminopurin-9-yl (i.e. adeninyl) group, a 6-aminopurin-9-yl group having an amino group protected with a protective group for synthesis of nucleic acid, a 2,6-diaminopurin-9-yl group, a 2-amino-6-chloropurin-9-yl group, a 2-amino-6-chloropurin-9-yl group having an amino group protected with a protective group for synthesis of nucleic acid, a 2-amino-6-fluoropurin-9-yl group, a 2-amino-6-fluoropurin-9-yl group having an amino group protected with a protective group for synthesis of nucleic acid, a 2-amino-6-bromopurin-9-yl group, a 2-amino-6-bromopurin-9-yl group having an amino group protected with a protective group for synthesis of nucleic acid, a 2-amino-6-hydroxypurin-9-yl (i.e. guaninyl) group, a 2-amino-6-hydroxypurin-9-yl group having an amino group protected with a protective group for synthesis of nucleic acid, a 6-amino-2-methoxypurin-9-yl group, a 6-amino-2-chloropurin-9-yl group, a 6-amino-2-fluoropurin-9-yl group, a 2,6-dimethoxypurin-9-yl group, a 2,6-dichloropurin-9-yl group, a 6-mercaptopurin-9-yl group, a 2-oxo-4-amino-1,2-dihydropyrimidin-1-yl (i.e. cytosinyl) group, a 2-oxo-4-amino-1,2-dihydropyrimidin-1-yl group having an amino group protected with a protective group for synthesis of nucleic acid, a 2-oxo-4-amino-5-fluoro-1,2-dihydropyrimidin-1-yl group, a 2-oxo-4-amino-5-fluoro-1,2-dihydropyrimidin-1-yl group having an amino group protected with a protective group for synthesis of nucleic acid, a 4-amino-2-oxo-5-chloro-1,2-dihydropyrimidin-1-yl group, a 2-oxo-4-methoxy-1,2-dihydropyrimidin-1-yl group, a 2-oxo-4-mercapto-1,2-dihydropyrimidin-1-yl group, a 2-oxo-4-hydroxy-1,2-dihydropyrimidin-1-yl (i.e. uracinyl) group, a 2-oxo-4-hydroxy-5-methyl-1,2-dihydropyrimidin-1-yl (i.e. thyminyl) group, a 4-amino-5-methyl-2-oxo-1,2-dihydropyrimidin-1-yl (i.e. 5-methylcytosinyl) group, or a 4-amino-5-methyl-2-oxo-1,2-dihydropyrimidin-1-yl group having an amino group protected with a protective group for synthesis of nucleic acid.
